# EUROPÄISCHE PATENTSCHRIFT

(11) **EP 1 088 082 B1**
(45) Veröffentlichungstag und Bekanntmachung des Hinweises auf die Patenterteilung: **17.08.2005**
(21) Anmeldenummer: 99950355.0
(22) Anmeldetag: 07.05.1999
(51) Int. Cl.: C12N 15/56, C12N 9/44, C12N 15/82, C12N 1/21, C12N 5/10, A01H 5/00, A01H 5/10, C08B 30/00

(54) **NUCLEINSÄUREMOLEKÜLE CODIEREND ENZYME AUS WEIZEN, DIE AN DER STÄRKESYNTHESE BETEILIGT SIND**
NUCLEIC ACID MOLECULES WHICH CODE FOR ENZYMES DERIVED FROM WHEAT AND WHICH ARE INVOLVED IN THE SYNTHESIS OF STARCH
MOLECULES D'ACIDE NUCLEIQUE CODANT POUR DES ENZYMES ISSUES DU FROMENT ET PARTICIPANT A LA SYNTHESE DE L'AMIDON

(30) Priorität: 08.05.1998 DE 19820608
(43) Veröffentlichungstag der Anmeldung: 04.04.2001
(73) Patentinhaber: Bayer CropScience GmbH, 65929 Frankfurt/Main (DE)
(72) Erfinder: LÖRZ, Horst, D-22589 Hamburg (DE); LÜTTICKE, Stephanie, D-20099 Hamburg (DE); ABEL, Gernot, D-20146 Hamburg (DE); GENSCHEL, Ulrich, D-22769 Hamburg (DE)
(74) Vertreter: VOSSIUS & PARTNER
(86) Internationale Anmeldenummer: PCT/EP1999/003141
(87) Internationale Veröffentlichungsnummer: WO 1999/058690

(56) Entgegenhaltungen:
- WO-A-96/03513
- WO-A-99/14314
- JAMES M G ET AL: "CHARACTERIZATION OF THE MAIZE GENE SUGARY1, A DETERMINANT OF STARCHCOMPOSITION IN KERNELS" PLANT CELL,US,AMERICAN SOCIETY OF PLANT PHYSIOLOGISTS, ROCKVILLE, MD, Bd. 7, Seite 417-429 XP002033602 ISSN: 1040-4651 in der Anmeldung erwähnt
- JAMES ET AL: "Zea mays Su1p (Sugary1) mRNA, partial cds" EMBL NUCLEOTIDE SEQUENCE, U18908, 19. April 1995 (1995-04-19), XP002084161

## Beschreibung

Die vorliegende Erfindung betrifft Nucleinsäuremoleküle, die ein Enzym aus Weizen codieren, das an der Stärkesynthese in Pflanzen beteiligt ist. Bei diesem Enzym handelt es sich um eine Isoamylase.

Weiterhin betrifft die Erfindung Vektoren, Wirtszellen, sowie Pflanzenzellen und Pflanzen, die die erfindungsgemäßen Nucleinsäuremolekülen enthalten.

Ferner werden Verfahren zur Herstellung transgener Pflanzen beschrieben, die aufgrund der Einführung von erfindungsgemäßen Nucleinsäuremolekülen eine in ihren Eigenschaften veränderte Stärke synthetisieren.

Im Hinblick auf die zunehmende Bedeutung, die pflanzlichen Inhaltsstoffen als erneuerbaren Rohstoffquellen in letzter Zeit beigemessen wird, ist es eine der Aufgaben der biotechnologischen Forschung, sich um eine Anpassung dieser pflanzlichen Rohstoffe an die Anforderungen der verarbeitenden Industrie zu bemühen. Um eine Anwendung von nachwachsenden Rohstoffen in möglichst vielen Einsatzgebieten zu ermöglichen, ist es darüber hinaus erforderlich, eine große Stoffvielfalt zu erreichen.

Neben Ölen, Fetten und Proteinen stellen Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in höheren Pflanzen ist. Hierbei ist Weizen eine der wichtigsten Kulturpflanzen, da ca. 20 % der Gesamtstärkeproduktion der Europäischen Gemeinschaft aus ihr gewonnen werden.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades des Auftretens von Verzweigungen der Glucoseketten und deren Kettenlängen unterscheiden, die darüber hinaus derivatisiert, z.B. phosphoryliert sein können. Daher stellt Stärke keinen einheitlichen Rohstoff dar. Man unterscheidet insbesondere die Amylosestärke, ein im wesentlichen unverzweigtes Polymer aus alpha-1,4-glycosidisch verknüpften Glucosemolekülen, von der Amylopektin-Stärke, die ihrerseits ein komplexes Gemisch aus unterschiedlich verzweigten Glucoseketten darstellt. Die Verzweigungen kommen dabei durch das Auftreten von zusätzlichen alpha-1,6-glycosidischen Verknüpfungen zustande. In Weizen besteht die synthetisierte Stärke zu ca. 11 bis 37 % aus Amylose-Stärke.

Um eine möglichst vielfältige Anwendung von geeigneten Stärken für unterschiedlichste industrielle Bedürfnisse zu ermöglichen, ist es wünschenswert, Pflanzen zur Verfügung zu stellen, die in der Lage sind, modifizierte Stärken zu synthetisieren, die für verschiedene Verwendungszwecke besonders gut geeignet sind. Eine Möglichkeit, derartige Pflanzen bereitzustellen, besteht in züchterischen Maßnahmen. Die züchterischen Einflußnahme erweist sich beim Weizen aufgrund seines polyploiden Charakters (tetra- und hexaploid) jedoch sehr schwer. Erst kürzlich gelang durch Kreuzung natürlich auftretender Mutanten die Herstellung eines "waxy" (Amylose-freien) Weizens (Nakamura et al., Mol. Gen. Genet. 248 (1995), 253-259).

Eine Alternative zu züchterischen Verfahren besteht in der gezielten Modifikation stärkeproduzierender Pflanzen durch gentechnologische Methoden. Voraussetzung hierfür ist jedoch die Identifizierung und Charakterisierung der an der Stärkesynthese und/oder Stärkemodifikation beteiligten Enzyme sowie die Isolierung der diese Enzyme codierenden Nucleinsäuremoleküle.

Die biochemischen Synthesewege, die zum Aufbau von Stärke führen, sind im wesentlichen bekannt. Die Stärkesynthese in pflanzlichen Zellen findet in den Plastiden statt. In photosynthetisch aktiven Geweben sind dies die Chloroplasten, in photosynthetisch inaktiven, stärkespeichernden Geweben die Amyloplasten.

Für eine weitere gezielte Veränderung des Verzweigungsgrades von in Pflanzen synthetisierter Stärke mit Hilfe gentechnischer Verfahren ist es nach wie vor erforderlich, DNA-Sequenzen zu identifizieren, die Enzyme codieren, die am Stärkemetabolismus, insbesondere an der Einführung oder am Abbau von Verzweigungen innerhalb der Stärkemoleküle, beteiligt sind.

Neben den sog. Q-Enzymen, die Verzweigungen in Stärkemoleküle einführen, kommen in Pflanzen Enzyme vor, die Verzweigungen auflösen können. Diese Enzyme werden als Debranching-Enzyme bezeichnet und werden entsprechend ihrer Substratspezifität in drei Gruppen eingeteilt:
(a) Die Pullulanasen, die neben Pullulan auch Amylopektin als Substrat nutzen, kommen in Mikroorganismen, z.B. *Klebsiella,* und in Pflanzen vor. In Pflanzen werden diese Enzyme auch als R-Enzyme bezeichnet.
(b) Die Isoamylasen, die nicht Pullulan, wohl aber Glycogen und Amylopektin als Substrat nutzen, kommen ebenfalls in Mikroorganismen und Pflanzen vor. Isoamylasen wurden beispielsweise in Mais (Manners & Carbohydr. Res. 9 (1969), 107) und Kartoffel (Ishizaki et al., Agric. Biol. Chem. 47 (1983), 771-779) beschrieben.
(c) Die Amylo-1,6-Glucosidasen sind in Säugern und Hefen beschrieben und nutzen Grenzdextrine als Substrat.

In Zuckerrüben konnte von U et al. (Plant Physiol. 98 (1992), 1277-1284) neben fünf Endo-und zwei Exoamylasen nur ein Debranching-Enzym vom Pullulanase-Typ nachgewiesen werden. Dieses Enzym, das eine Größe von ca. 100 kD und ein pH- Optimum von 5,5 aufweist, ist in den Chloroplasten lokalisiert. Auch für Spinat wurde ein Debranching-Enzym beschrieben, das Pullulan als Substrat verwendet. Sowohl das Debranching-Enzym aus Spinat als auch das aus der Zuckerrübe besitzen bei der Reaktion mit Amylopektin als Substrat verglichen mit Pullulan als Substrat, eine 5-fach geringere Aktivität (Ludwig et al., Plant Physiol. 74 (1984), 856-861; Li et al., Plant Physiol. 98 (1992), 1277-1284).

Bei der landwirtschaftlich wichtigen stärkespeichernden Kulturpflanze Kartoffel wurde die Aktivität eines Debranching-Enzyms von Hobson et al. (J. Chem. Soc., (1951), 1451) untersucht. Es gelang der Nachweis, daß das entsprechende Enzym im Gegensatz zum Q-Enzym keine kettenverlängernde Aktivität besitzt, sondern lediglich alpha-1,6-glycosidische Bindungen hydrolysiert. Das Enzym konnte jedoch bisher nicht genauer charakterisiert werden. Im Fall der Kartoffel wurden bereits Verfahren zur Reinigung des Debranching-Enzyms sowie partielle Peptidsequenzen des gereinigten Proteins vorgeschlagen (WO 95/04826). Für Spinat wurde inzwischen die Reinigung eines Debranching-Enzyms sowie die Isolierung einer entsprechenden cDNA beschrieben (Renz et al., Plant Physiol. 108 (1995), 1342).

Für Mais wurde bisher in der Literatur lediglich die Existenz eines Debranching-Enzyms beschrieben. Dieses wird aufgrund seiner Substratspezifität in die Gruppe der Isoamylasen eingeordnet (siehe z.B. Hannah et al., Scientia Horticulturae 55 (1993), 177-197 oder Garwood (1984) in Starch Chemistry and Technology, Whistler, R.L., BeMiller, J.N., Puschall, E.F. (eds.), Academic Press San Diego, New York, Boston, 25-86). Die entsprechende Mutante wird als sugary bezeichnet. Das Gen des sugary-Locus wurde kürzlich cloniert (siehe James et al., Plant Cell 7 (1995), 417-429). Neben dem sugary-Locus ist bisher in Mais kein anderer Genlocus bekannt, der ein Protein mit Debranching-Enzymaktivität codiert. Es gab bisher auch keinerlei Hinweise, daß andere Debranching-Enzymformen in Mais vorkommen. Will man transgene Maispflanzen herstellen, die keinerlei Debranching-Enzymaktivitäten mehr aufweisen, z.B. um eine Verlängerung des Verzweigungsgrades der Amylopektinstärke zu erzielen, so ist es erforderlich, alle in Mais vorkommenden Debranching-Enzymformen zu identifizieren und die entsprechenden Gene oder cDNA-Sequenzen zu isolieren.

Um weitere Möglichkeiten bereitzustellen, beliebige stärke-speichernde Pflanzen, vorzugsweise Getreide, insbesondere Weizen, dahingehend zu verändern, daß sie eine modifizierte Stärke synthesieren, ist es erforderlich, jeweils DNA-Sequenzen zu identifizieren, die weitere Isoformen von Verzweigungsenzymen codieren.

Der vorliegenden Erfindung liegt somit die Aufgabe zugrunde, Nucleinsäuremoleküle zur Verfügung zu stellen, die an der Stärkebiosynthese beteiligte Enzyme codieren und mit deren Hilfe es möglich ist, gentechnisch modifizierte Pflanzen herzustellen, die die Herstellung von in ihren chemischen und/oder physikalischen Eigenschaften veränderten pflanzlichen Stärken ermöglichen.

Diese Aufgabe wird durch die Bereitstellung der in den Patentansprüchen bezeichneten Ausführungsformen gelöst.

Die vorliegende Erfindung betrifft daher ein Nucleinsäuremolekül, das ein Protein mit der Funktion einer Isoamylase aus Weizen codiert, vorzugsweise ein Protein, das durch die unter Seq ID No. 3 angegebene Aminosäuresequenz definiert ist. Insbesondere betrifft die Erfindung ein Nucleinsäuremolekül, das die unter Seq ID No. 2 angegebene Nucleotidsequenz oder einen Teil davon enthält, bevorzugt ein Molekül, das die in Seq ID No. 2 angegebene codierende Region enthält sowie hierzu entsprechende (korrespondierende) Ribonucleotidsequenzen. Ganz besonders bevorzugt ist ein Nucleinsäuremolekül, das desweiteren regulatorische Elemente enthält, die die Transkription sowie gegebenenfalls die Translation der besagten Nucleinsäuremoleküle gewährleistet.
Ferner betrifft die vorliegende Erfindung ein Nucleinsäuremolekül, das mit einem der erfindungsgemäßen Nukleinsäuremoleküle hybridisiert.

Gegenstand der Erfindung ist ebenfalls ein Nucleinsäuremolekül, das eine Isoamylase aus Weizen codiert und dessen Sequenz aufgrund der Degeneration des genetischen Codes von den Nucleotidsequenzen der oben beschriebenen Moleküle abweicht.

Die Erfindung betrifft auch ein Nucleinsäuremolekül, das eine Sequenz aufweist, die zu der gesamten oder einem Teil einer der obengenannten Sequenzen komplementär ist.

Der Begriff "Hybridisierung" bedeutet im Rahmen dieser Erfindung eine Hybridisierung unter konventionellen Hybridisierungsbedingungen, vorzugsweise unter stringenten Bedingungen, wie sie beispielsweise in Sambrook et al., Molecular Cloning, A Laboratory Manual, 2. Aufl. (1989) Cold Spring Harbor Laboratory Press, Cold Spring Harbor, NY) beschrieben sind.

### Besonders bevorzugt erfolgt eine "Hybridisierung" unter den folgenden Bedingungen:

- Hybridisierungspuffer:: 2 x SSC; 10 x Denhardt-Lösung (Fikoll 400 + PEG + BSA; Verhältnis 1:1:1); 0,1% SDS; 5 mM EDTA; 50 mM Na₂HPO₄; 250 µg/ml Heringssperma-DNA; 50 µg/ml tRNA; oder 0,25 M Natriumphosphatpuffer pH 7,2; 1 mM EDTA; 7% SDS
- Hybridisierungstemperatur: T = 65 bis 70°C
- Waschpuffer:: 0,2 x SSC; 0,1 % SDS
- Waschtemperatur: T = 40 bis 75°C.

Nucleinsäuremoleküle, die mit den erfindungsgemäßen Nucleinsäuremolekülen hybridisieren, können prinzipiell Isoamylasen aus jeder beliebigen Weizenpflanze codieren, die derartige Proteine exprimiert.

Nucleinsäuremoleküle, die mit den erfindungsgemäßen Molekülen hybridisieren, können z.B. aus genomischen oder aus cDNA-Bibliotheken von Weizen oder Weizenpflanzengewebe isoliert werden. Alternativ können sie durch gentechnische Methoden oder durch chemische Synthese hergestellt werden.

Die Identifizierung und Isolierung derartiger Nucleinsäuremoleküle kann dabei unter Verwendung der erfindungsgemäßen Moleküle oder Teile dieser Moleküle bzw. der reversen Komplemente dieser Moleküle erfolgen, z.B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY).

Als Hybridisierungsprobe können z.B. Nucleinsäuremoleküle verwendet werden, die exakt die oder im wesentlichen die unter Seq ID No. 2 angegebene Nucleotidsequenz oder Teile dieser Sequenzen aufweisen. Bei den als Hybridisierungsprobe verwendeten Fragmenten kann es sich auch um synthetische Fragmente handeln, die mit Hilfe der gängigen Synthesetechniken hergestellt wurden und deren Sequenz im wesentlichen mit der eines erfindungsgemäßen Nucleinsäuremoleküls übereinstimmt.

Die mit den erfindungsgemäßen Nucleinsäuremolekülen hybridisierenden Moleküle umfassen auch Fragmente, Derivate und allelische Varianten der oben beschriebenen Nucleinsäuremoleküle, die eine erfindungsgemäße Isoamylase aus Weizen codieren. Unter Fragmenten werden dabei Teile der Nucleinsäuremoleküle verstanden, die lang genug sind, um eines der beschriebenen Proteine zu codieren. Der Ausdruck Derivat bedeutet in diesem Zusammenhang, daß die Sequenzen dieser Moleküle sich von den Sequenzen der oben beschriebenen Nucleinsäuremoleküle an einer oder mehreren Positionen unterscheiden und einen hohen Grad an Homologie zu diesen Sequenzen aufweisen. Homologie bedeutet dabei eine Sequenzidentität von über 90%. Die Abweichungen zu den oben beschriebenen Nucleinsäuremolekülen können dabei durch Deletion, Substitution, Insertion oder Rekombination entstanden sein.

Homologie bedeutet ferner, daß funktionelle und/oder strukturelle Äquivalenz zwischen den betreffenden Nucleinsäuremolekülen oder den durch sie codierten Proteinen, besteht. Bei den Nucleinsäuremolekülen, die homolog zu den oben beschriebenen Molekülen sind und Derivate dieser Moleküle darstellen, handelt es sich in der Regel um Variationen dieser Moleküle, die Modifikationen darstellen, die diselbe biologische Funktion ausüben. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln als auch um synthetisch hergestellte oder durch rekombinante DNA-Techniken erzeugte Varianten.

Die von den verschiedenen Varianten der erfindungsgemäßen Nucleinsäuremoleküle codierten Isoamylasen weisen bestimmte gemeinsame Charakteristika auf. Dazu können z.B. Enzymaktivität, Molekulargewicht, immunologische Reaktivität, Konformation etc. gehören sowie physikalische Eigenschaften wie z.B. das Laufverhalten in Gelelektrophoresen, chromatographisches Verhalten, Sedimentationskoeffizienten, Löslichkeit, spektroskopische Eigenschaften, Ladungseigenschaften, Stabilität; pH-Optimum, Temperatur-Optimum etc.

Bei dem durch die erfindungsgemäßen Nucleinsäuremoleküle codierten Protein handelt es sich um eine Isoamylase aus Weizen. Diese Proteine weisen gewisse Homologiebereiche zu bisher bekannten Isoamylasen aus anderen Pflanzenarten auf.

Bei den erfindungsgemäßen Nucleinsäuremolekülen kann es sich um DNA-Moleküle handeln, insbesondere um cDNA- oder genomische Moleküle. Ferner können die erfindungsgemäßen Nucleinsäuremoleküle RNA-Moleküle sein, die z.B. durch Transkription eines erfindungsgemäßen Nucleinsäuremoleküls resultieren können. Die erfindungsgemäßen Nucleinsäuremoleküle können z. B. aus natürlichen Quellen gewonnen sein oder durch rekombinante Techniken oder synthetisch hergestellt sein.

Gegenstand der Erfindung sind auch Oligonucleotide, die spezifisch mit einem erfindungsgemäßen Nucleinsäuremolekül hybridisieren. Derartige Oliganucleotide haben eine Länge von mindestens 15 und vorzugsweise von mindestens 50 Nucleotiden. Die erfindungsgemäßen Oligonucleotide sind dadurch gekennzeichnet, dass sie spezifisch mit erfindungsgemäßen Nucleinsäuremolekülen hybridisieren, d.h. nicht oder nur in sehr geringem Ausmaß mit Nucleinsäuresequenzen, die andere Proteine, insbesondere andere Isoamylasen codieren. Die erfindungsgemäßen Oligonucleotide können beispielsweise als Primer für eine PCR-Reaktion verwendet werden oder als Hybridisierungsprobe für die Isolierung verwandter Gene. Ebenso können sie Bestandteile von antisense-Konstrukten sein oder von DNA-Molekülen, die für geeignete Ribozyme codieren.

Ferner betrifft die Erfindung Vektoren, insbesondere Plasmide, Cosmide, Phagemide, Viren, Bacteriophagen und andere in der Gentechnik gängige Vektoren, die die oben beschriebenen erfindungsgemäßen Nucleinsäuremoleküle enthalten. Derartige Vektoren sind zur Transformation pro- oder eukaryontischer, vorzugsweise pflanzlicher Zellen geeignet.

Besonders bevorzugt erlauben die Vektoren die Integration der erfindungsgemäßen Nucleinsäuremoleküle, gegebenenfalls zusammen mit flankierenden regulatorischen Regionen, in das Genom der Pflanzenzelle. Beispiele hierfür sind binäre Vektoren, die bei dem Agrobakterien-vermittelten Gentransfer eingesetzt werden können. Vorzugsweise ist durch die Integration eines erfindungsgemäßen Nucleinsäuremoleküls in sense- oder anti-sense-Orientierung die Synthese einer translatierbaren oder gegebenenfalls nicht-translatierbaren RNA in den transformierten pro- oder eukaryontischen Zellen gewährleistet.

Der Begriff "Vektor" bezeichnet im allgemeinen ein geeignetes, dem Fachmann bekanntes Hilfsmittel, das den gezielten Transfer eines ein- oder doppelsträngigen Nukleinsäuremoleküls in eine Wirtszelle ermöglicht, beispielsweise einen DNA- oder RNA-Virus, ein Virusfragment, ein Plasmidkonstrukt, das in An- oder Abwesenheit von regulatorischen Elementen zum Nukleinsäure-Transfer in Zellen geeignet sein kann, Trägermaterialien wie Glasfaser oder auch Metallpartikel wie sie z.B. beim "particle gun"-Verfahren eingesetzt werden können, er kann aber auch ein Nukleinsäuremolekül umfassen, das durch chemische oder physikalische Verfahren direkt in eine Zelle gebracht werden kann.

In einer bevorzugten Ausführungsform sind die in den Vektoren enthaltenen Nucleinsäuremoleküle verknüpft mit regulatorischen Elementen, die die Transkription und Synthese einer translatierbaren RNA in pro- oder eukaryontischen Zellen gewährleisten oder - sofern gewünscht - die Synthese einer nicht-translatierbaren RNA gewährleisten.

Die Expression der erfindungsgemäßen Nucleinsäuremoleküle in prokaryontischen Zellen, beispielsweise in Escherichia coli, ist für eine genauere Charakterisierung der enzymatischen Aktivitäten der Enzyme, für die diese Moleküle codieren, von Bedeutung. Es ist insbesondere möglich, das Produkt, das von den entsprechenden Enzymen in Abwesenheit anderer, in der pflanzlichen Zelle an der Stärkesynthese beteiligter Enzyme synthetisiert wird, zu charakterisieren. Dies läßt Rückschlüsse zu auf die Funktion, die das entsprechende Protein bei der Stärkesynthese in der Pflanzenzelle ausübt.

Darüber hinaus ist es möglich, mittels gängiger molekularbiologischer Techniken (siehe z.B. Sambrook et al., 1989, Molecular Cloning, A Laboratory Manual, 2. Aufl. Cold Spring Harbor Laboratory Press, Cold Spring Harbor, NY) verschiedenartige Mutationen in die erfindungsgemäßen Nucleinsäuremoleküle einzuführen, wodurch es zur Synthese von Proteinen mit eventuell veränderten biologischen Eigenschaften kommt. Hierbei ist zum einen die Erzeugung von Deletionsmutanten möglich, bei denen durch fortschreitende Deletionen vom 5'- oder vom 3'-Ende der codierenden DNA-Sequenz Nucleinsäuremoleküle erzeugt werden, die zur Synthese entsprechend verkürzter Proteine führen. Durch derartige Deletionen am 5'-Ende der Nucleotidsequenz ist es beispielsweise möglich, Aminosäuresequenzen zu identifizieren, die für die Translokation des Enzyms in die Plastiden verantwortlich sind (Transitpeptide). Dies erlaubt es, gezielt Enzyme herzustellen, die durch Entfernen der entsprechenden Sequenzen nicht mehr in den Plastiden, sondern im Cytosol lokalisiert sind, oder aufgrund der Addition von anderen Signalsequenzen in anderen Kompartimenten lokalisiert sind.

Andererseits ist auch die Einführung von Punktmutationen denkbar an Positionen, bei denen eine Veränderung der Aminosäuresequenz einen Einfluß beispielweise auf die Enzymaktivität oder die Regulierung des Enzyms hat. Auf diese Weise können z.B. Mutanten hergestellt werden, die einen veränderten Kₘ-Wert besitzen oder nicht mehr den normalerweise in der Zelle vorliegenden Regulationsmechanismen über alloste-rische Regulation oder kovalente Modifizierung unterliegen.

Des weiteren können Mutanten hergestellt werden, die eine veränderte Substrat- oder Produktspezifität des erfindungsgemäßen Proteins aufweisen. Weiterhin können Mutanten hergestellt werden, die ein verändertes Aktivitäts-TemperaturProfil des erfindungsgemäßen Proteins aufweisen.

Für die gentechnische Modifikation prokaryontischer Zellen können die erfindungsgemäßen Nucleinsäuremoleküle oder Teile dieser Moleküle in Plasmide eingebracht werden, die eine Mutagenese oder eine Sequenzveränderung durch Rekombination von DNA-Sequenzen erlauben. Mit Hilfe von Standardverfahren (vgl. Sambrook et al., 1989, Molecular Cloning: A laboratory manual, 2. Aufl., Cold Spring Harbor Laboratory Press, NY, USA) können Basenaustausche vorgenommen oder natürliche oder synthetische Sequenzen hinzugefügt werden. Für die Verbindung der DNA-Fragmente untereinander können an die Fragmente Adaptoren oder Linker angesetzt werden. Ferner können Manipulationen, die passende Restriktionsschnittstellen zur Verfügung stellen oder die überflüssige DNA oder Restriktionsschnittstellen entfernen, eingesetzt werden. Wo Insertionen, Deletionen oder Substitutionen in Frage kommen, können in vitro-Mutagenese, "primer repair", Restriktion oder Ligation verwendet werden. Als Analysemethode werden im allgemeinen Sequenzanalyse, Restriktionsanalyse oder weitere biochemisch-molekularbiologische Methoden durchgeführt.

In einer weiteren Ausführungsform betrifft die Erfindung Wirtszellen, insbesondere pro- oder eukaryontische Zellen, die mit einem oben beschriebenen erfindungsgemäßen Nucleinsäuremolekül oder einem erfindungsgemäßen Vektor transformiert sind, sowie Zellen, die von derart transformierten Zellen abstammen und ein erfindungsgemäßes Nucleinsäuremolekül oder einen Vektor enthalten. Dabei handelt es sich vorzugsweise um pro- oder eukaryontische Zellen, insbesondere um pflanzliche Zellen.

Gegenstand der Erfindung sind ferner rekombinant herstellbare Proteine mit der Aktivität einer Isoamylase, die durch die erfindungsgemäßen Nucleinsäuremoleküle codiert werden, sowie Verfahren zu deren Herstellung, worin eine erfindungsgemäße Wirtszelle unter dem Fachmann bekannten, geeigneten Bedingungen kultiviert wird, die die Synthese des erfindungsgemäßen Proteins erlauben, und es anschließend aus den Wirtszellen und/oder dem Kulturmedium isoliert wird.

Durch die Bereitstellung der erfindungsgemäßen Nucleinsäuremoleküle ist es nun möglich, mit Hilfe gentechnischer Methoden in den Stärkemetabolismus von Pflanzen gezielt einzugreifen und ihn dahingehend zu verändern, daß es zur Synthese einer modifizierten Stärke kommt, die in ihren physikalisch-chemischen Eigenschaften, beispielsweise dem Amylose/Amylopektin-Verhältnis, dem Verzweigungsgrad, der durchschnittlichen Kettenlänge, dem Phosphatgehalt, dem Verkleisterungsverhalten, den Gel- oder Filmbildungseigenschaften, der Stärkekorngröße und/oder der Stärkekornform im Vergleich zu bekannter Stärke verändert ist.

Möglich ist somit die Expression der erfindungsgemäßen Nucleinsäuremoleküle in pflanzlichen Zellen, um die Aktivität der entsprechenden Isoamylase zu erhöhen, oder die Einführung in Zellen, die dieses Enzym natürlicherweise nicht exprimieren. Durch die Expression der erfindungsgemäßen Nucleinsäuremoleküle besteht auch die Möglichkeit, die natürlicherweise bestehende Aktivität der erfindungsgemäßen Isoamylase in den pflanzlichen Zellen zu erniedrigen. Ferner ist es möglich, die erfindungsgemäßen Nucleinsäuremoleküle nach dem Fachmann bekannten Methoden zu modifizieren, um erfindungsgemäße Isoamylasen zu erhalten, die nicht mehr den natürlichen zelleigenen Regulationsmechanismen unterliegen, bzw. veränderte Temperatur-Aktivitätsprofile oder Substrat- bzw. Produktspezifitäten aufweisen.

Bei der Expression der erfindungsgemäßen Nucleinsäuremoleküle in Pflanzen besteht grundsätzlich die Möglichkeit, daß das synthetisierte Protein in jedem beliebigen Kompartiment der pflanzlichen Zelle lokalisiert sein kann. Um die Lokalisation in einem bestimmten Kompartiment zu erreichen, muß die die Lokalisation in Plastiden gewährleistende Sequenz deletiert werden und die verbleibende codierende Region gegebenenfalls mit DNA-Sequenzen verknüpft werden, die die Lokalisierung in dem jeweiligen Kompartiment gewährleisten. Derartige Sequenzen sind bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die vorliegende Erfindung betrifft somit auch ein Verfahren zur Herstellung transgener Pflanzenzellen, die mit einem erfindungsgemäßen Nucleinsäuremolekül oder Vektor transformiert werden, worin ein erfindungsgemäßes Nucleinsäuremolekül oder ein erfindungsgemäßer Vektor in das Genom einer pflanzlichen Zelle integriert wird, die transgenen Pflanzenzellen, die mittels eines erfindungsgemäßen Nucleinsäuremoleküls oder Vektors transformiert wurden sowie transgene Pflanzenzellen, die von derartig transformierten Zellen abstammen. Die erfindungsgemäßen Zellen enthalten ein oder mehrere erfindungsgemäße Nucleinsäuremoleküle oder Vektoren, wobei diese vorzugsweise mit regulatorischen DNA-Elementen verknüpft sind, die die Transkription in pflanzlichen Zellen gewährleisten, insbesondere mit einem geeigneten Promotor. Derartige Zellen lassen sich von natürlicherweise vorkommenden Pflanzenzellen unter anderem dadurch unterscheiden, daß sie ein erfindungsgemäßes Nucleinsäuremolekül enthalten, das natürlicherweise in diesen Zellen nicht vorkommt oder dadurch, daß ein solches Molekül an einem Ort im Genom der Zelle integriert vorliegt, an dem es sonst nicht vorkommt, d.h. in einer anderen genomischen Umgebung. Ferner lassen sich derartige erfindungsgemäße transgene Pflanzenzellen von natürlicherweise vorkommenden Pflanzenzellen dadurch unterscheiden, daß sie mindestens eine Kopie eines erfindungsgemäßen Nucleinsäuremoleküls stabil integriert in ihr Genom enthalten, gegebenenfalls zusätzlich zu natürlicherweise in den Zellen vorkommenden Kopien eines solchen Moleküls. Handelt es sich bei dem (den) in die Zellen eingeführten Nucleinesäuremolekül(en) um zusätzliche Kopien zu bereits natürlicherweise in den Zellen vorkommenden Molekülen, so lassen sich die erfindungsgemäßen Pflanzenzellen von natürlicherweise vorkommenden insbesondere dadurch unterscheiden, daß diese zusätzliche(n) Kopie(n) an Orten im Genom lokalisiert ist/sind an denen sie natürlicherweise nicht vorkommt (vorkommen). Dies läßt sich beispielsweise mit Hilfe einer Southern Blot-Analyse nach dem Fachmann bekannten Verfahren einfach überprüfen.

Ist das in das pflanzliche Genom eingeführte erfindungsgemäße Nucleinsäuremolekül heterolog in Bezug auf die Pflanzenzelle, so weisen die transgenen Pflanzenzellen Transkripte der erfindungsgemäßen Nucleinsäuremoleküle auf, die sich z. B. durch Northern-Blot-Analyse nach dem Fachmann bekannten Verfahren einfach nachweisen lassen.

Ist das eingeführte erfindungsgemäße Nucleinsäuremolekül homolog in Bezug auf die Pflanzenzelle, können die erfindungsgemäßen Zellen von natürlicherweise auftretenden beispielsweise aufgrund der zusätzlichen Expression erfindungsgemäßer Nucleinsäuremoleküle unterschieden werden. Die transgenen Pflanzenzellen enthalten vorzugsweise mehr Transkripte der erfindungsgemäßen Nucleinsäuremoleküle. Dies kann z. B. durch Northern-Blot-Analyse nachgewiesen werden. "Mehr" bedeutet dabei vorzugweise mindestens 10% mehr, bevorzugt mindestens 20% mehr und besonders bevorzugt mindestens 50% mehr Transkripte als entsprechende, nicht-transformierte Zellen. Vorzugsweise weisen die Zellen ferner eine entsprechende (mindestens 10%, 20% bzw. 50%ige) Aktivitätsteigerung oder ggf. eine Aktivitätsreduzierung des erfindungsgemäßen Proteins auf. Die transgenen Pflanzenzellen können nach dem Fachmann bekannten Techniken zu ganzen Pflanzen regeneriert werden.

Ebenfalls ist ein Gegenstand der vorliegenden Erfindung ein Verfahren zur Herstellung transgener Pflanzen, worin ein oder mehrere erfindungsgemäße Nucleinsäuremoleküle oder Vektoren in das Genom einer pflanzlichen Zelle integriert werden und eine vollständige Pflanze aus besagter Pflanzenzelle regeneriert wird. Ferner sind Gegenstand der Erfindung Pflanzen, die die oben beschriebenen transgenen Pflanzenzellen enthalten. Bei den transgenen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, vorzugsweise stärkesynthetisierende bzw. stärkespeichernde Pflanzen, besonders bevorzugt Roggen, Gerste Hafer, Weizen, Hirse, Sago, Mais, Reis, Erbse, Markerbse, Maniok, Kartoffel, Tomate, Raps, Sojabohne, Hanf, Flachs, Sonnenblume, Kuherbse oder Arrowroot, insbesondere Weizen, Mais, Reis und Kartoffel.

Die Erfindung betrifft ebenfalls Vermehrungsmaterial der erfindungsgemäßen Pflanzen, beispielsweise Früchte, Samen, Knollen, Wurzelstöcke, Sämlinge, Stecklinge, Calli, Protoplasten, Zellkulturen etc..

Die vorliegende Erfindung betrifft ferner ein Verfahren zur Herstellung einer modifizierten Stärke umfassend den Schritt der Extraktion der Stärke aus einer oben beschriebenen erfindungsgemäßen Pflanze und/oder aus stärkespeichernden Teilen einer solchen Pflanze.

Verfahren zur Extraktion der Stärke von Pflanzen oder von stärkespeichernden Teilen von Pflanzen, insbesondere aus Weizen, sind dem Fachmann bekannt, vgl. z.B. Eckhoff et al. (Cereal Chem. 73 (1996) 54-57) "Starch: Chemistry and Technology (Hrsg.: Whistler, BeMiller und Paschall (1994), 2. Ausgabe, Academic Press Inc. London Ltd; ISBN 0-12-746270-8; siehe z. B. Kapitel XII, Seite 412-468: Mais und Sorghum-Stärken: Herstellung; von Watson; Kapitel XIII, Seite 469-479: Tapioca-, Arrowroot- und Sagostärken: Herstellung; von Corbishley und Miller; Kapitel XIV, Seite 479-490: Kartoffelstärke: Herstellung und Verwendungen; von Mitch; Kapitel XV, Seite 491 bis 506: Weizenstärke: Herstellung, Modifizierung und Verwendungen; von Knight und Oson; und Kapitel XVI, Seite 507 bis 528: Reisstärke: Herstellung und Verwendungen; von Rohmer und Klem). Vorrichtungen, die für gewöhnlich bei Verfahren zur Extraktion von Stärke von Pflanzenmaterial verwendet werden, sind Separatoren, Dekanter, Hydrocyclone, Sprühtrockner und Wirbelschichttrockner.

Die erfindungsgemäßen transgenen Pflanzenzellen und Pflanzen synthetisieren aufgrund der Expression eines erfindungsgemäßen Nucleinsäuremoleküls eine Stärke, die in ihren physikalisch-chemischen Eigenschaften, z.B. dem Amylose/Amylopektin-Verhältnis, dem Verzweigungsgrad, der durchschnittlichen Kettenlänge, dem Phosphatgehalt, dem Verkleisterungsverhalten, der Stärkekorngröße und/oder der Stärkekornform im Vergleich zu in Wildtyp-Pflanzen synthetisierter Stärke verändert ist. Insbesondere kann eine solche Stärke im Hinblick auf die Viskosität und/oder die Film- oder Gelbildungseigenschaften von Kleistern dieser Stärke im Vergleich zu bekannten Stärken verändert sein.

Offenbart ist ferner eine Stärke, die aus den erfindungsgemäßen Pflanzenzellen, Pflanzen sowie deren Vermehrungsmaterial erhältlich ist und Stärke, die durch das oben beschriebene erfindungsgemäße Verfahren erhältlich ist.

Ferner ist es möglich, mit Hilfe der erfindungsgemäßen Nucleinsäuremoleküle Pflanzenzellen und Pflanzen zu erzeugen, bei denen die Aktivität eines erfindungsgemäßen Proteins verringert ist. Dies führt ebenfalls zur Synthese einer Stärke mit veränderten chemischen und/oder physikalischen Eigenschaften verglichen mit Stärke aus Wildtyp-Pflanzenzellen.

Ein weiterer Gegenstand der Erfindung ist somit auch ein transgene Pflanzenzelle, enthaltend ein erfindungsgemäßes Nukleinsäuremolekül, in der die Aktivität einer Isoamylase im Vergleich zu einer nichttransformierten Zellen reduziert ist.

Die Herstellung von Pflanzenzellen mit einer verringerten Aktivität einer Isoamylase kann beispielsweise erzielt werden durch die Expression einer entsprechenden antisense-RNA, einer sense-RNA zur Erzielung eines Cosuppressionseffektes oder die Expression eines entsprechend konstruierten Ribozyms, das spezifisch Transkripte spaltet, die eine Isoamylase codieren, unter Verwendung der erfindungsgemäßen Nucleinsäuremoleküle nach dem Fachmann bekannten Verfahren, vgl. Jorgensen (Trends Biotechnol. 8 (1990), 340-344), Niebel et al., (Curr. Top. Microbiol. Immunol. 197 (1995), 91-103), Flavell et al. (Curr. Top. Microbiol. Immunol. 197 (1995), 43-46), Palaqui und Vaucheret (Plant. Mol. Biol. 29 (1995), 149-159), Vaucheret et al., (Mol. Gen. Genet. 248 (1995), 311-317), de Borne et al. (Mol. Gen. Genet. 243 (1994), 613-621).

Vorzugsweise wird zur Reduzierung der Aktivität einer erfindungsgemäßen Isoamylase in den pflanzlichen Zellen die Anzahl der sie codierenden Transkripte reduziert, z.B. durch Expression einer antisense-RNA.

Hierzu kann zum einen ein DNA-Molekül verwendet werden, das die gesamte für ein erfindungsgemäßes Protein codierende Sequenz einschließlich eventuell vorhandener flankierender Sequenzen umfaßt, als auch DNA-Moleküle, die nur Teile der codierenden Sequenz umfassen, wobei diese Teile lang genug sein müssen, um in den Zellen einen antisense-Effekt zu bewirken. Es können im allgemeinen Sequenzen bis zu einer Mindestlänge von 15 bp, vorzugsweise einer Länge von 100-500 bp, für eine effiziente antisense-Inhibition insbesondere Sequenzen mit einer Länge über 500 bp verwendet werden. In der Regel werden DNA-Moleküle verwendet, die kürzer als 5000 bp, vorzugsweise Sequenzen, die kürzer als 2500 bp sind.

Möglich ist auch die Verwendung von DNA-Sequenzen, die einen hohen Grad an Homologie zu den Sequenzen der erfindungsgemäßen DNA-Moleküle aufweisen, aber nicht vollkommen identisch sind. Die minimale Homologie sollte größer als ca. 65 % sein. Die Verwendung von Sequenzen mit Homologien zwischen 95 und 100 % ist zu bevorzugen.

Offenbart ist auch ein Verfahren zur Herstellung einer modifizierten Stärke umfassend den Schritt der Extraktion der Stärke aus einer erfindungsgemäßen Zelle oder Pflanze und/oder aus stärkespeichernden Teilen einer solchen Pflanze.

Offenbart ist ferner Stärke, die aus den erfindungsgemäßen Zellen, Pflanzen sowie Vermehrungsmaterial oder deren Teilen erhältlich ist sowie Stärke, die durch ein erfindungsgemäßes Verfahren erhältlich ist.

Die Stärken können nach dem Fachmann bekannten Verfahren modifiziert werden und eignen sich in unmodifizierter oder modifizierter Form für verschiedene Verwendungen im Nahrungsmittel- oder Nicht-Nahrungsmittelbereich.

Grundsätzlich lassen sich die Einsatzmöglichkeiten der erfindungsgemäßen Stärken in zwei große Bereiche unterteilen. Der eine Bereich umfaßt die Hydrolyseprodukte der Stärke, hauptsächlich Glucose und Glucanbausteine, die über enzymatische oder chemische Verfahren erhalten werden. Sie dienen als Ausgangsstoff für weitere chemische Modifikationen und Prozesse, wie Fermentation. Für eine Reduktion der Kosten kann hierbei die Einfachheit und kostengünstige Ausführung eines Hydrolyseverfahrens von Bedeutung sein.

Gegenwärtig verläuft es im wesentlichen enzymatisch unter Verwendung von Amyloglucosidase. Vorstellbar wäre eine Kosteneinsparung durch einen geringeren Einsatz von Enzymen. Eine Strukturveränderung der Stärke, z.B. Oberflächenvergrößerung des Korns, leichtere Verdaulichkeit durch z.B. geringeren Verzweigungsgrad oder eine sterische Struktur, die die Zugänglichkeit für die eingesetzten Enzyme begrenzt, könnte dies bewirken.

Der andere Bereich, in dem die erfindungsgemäßen Stärken wegen ihrer polymeren Struktur als sogenannte native Stärke verwendet wird, gliedert sich in zwei weitere Einsatzgebiete:
1. Nahrungsmittelindustrie
   Stärke ist ein klassischer Zusatzstoff für viele Nahrungsmittel, bei denen sie im wesentlichen die Funktion des Bindens von wäßrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell- und Verkleisterungstemperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Taustabilität, die Viskositätsstabilität in Salzlösungen, die Verdaulichkeit sowie die Fähigkeit zur Komplexbildung mit z.B. anorganischen oder organischen Ionen.
2. Nicht-Nahrungmittelindustrie
   In diesem großen Bereich kann die Stärke als Hilfsstoff für unterschiedliche Herstellungsprozesse bzw. als Zusatzstoff in technischen Produkten eingesetzt werden. Bei der Verwendung der Stärke als Hilfsstoff ist hier insbesondere die Papier- und Pappeindustrie zu nennen.
   Die Stärke dient dabei in erster Linie zur Retardation (Zurückhaltung von Feststoffen), der Abbindung von Füllstoff- und Feinstoffteilchen, als Festigungsstoff und zur Entwässerung. Darüber hinaus werden die günstigen Eigenschaften der Stärke in bezug auf die Steifigkeit, die Härte, den Klang, den Griff, den Glanz, die Glätte, die Spaltfestigkeit sowie die Oberflächen ausgenutzt.
2.1 Papier- und Pappeindustrie
   Innerhalb des Papierherstellungsprozesses sind vier Anendungsbereiche, nämlich Oberfläche, Strich, Masse und Sprühen, zu unterscheiden. Die Anforderungen an die Stärke in bezug auf die Oberflächenbehandlung sind im wesentlichen ein hoher Weißegrad, eine angepaßte Viskosität, eine hohe Viskositätsstabilität, eine gute Filmbildung sowie eine geringe Staubbildung. Bei der Verwendung im Strich spielt der Feststoffgehalt, eine angepaßte Viskosität, ein hohes Bindevermögen sowie eine hohe Pigmentaffinität eine wichtige Rolle. Als Zusatz zur Masse ist eine rasche, gleichmäßige, verlustfreie Verteilung, eine hohe mechanische Stabilität und eine vollständige Zurückhaltung im Papierfließ von Bedeutung. Beim Einsatz der Stärke im Sprühbereich sind ebenfalls ein angepaßter Feststoffgehalt, hohe Viskosität sowie ein hohes Bindevermögen von Bedeutung.
2.2 Klebstoffindustrie
   Ein großer Einsatzbereich der Stärken besteht in der Klebstoffindustrie, wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90 % der Klebstoffe auf Stärkebasis werden in den Bereichen Wellpappenherstellung, Herstellung von Papiersäcken, Beuteln und Tüten, Herstellung von Verbundmaterialien für Papier und Aluminium, Herstellung von Kartonagen und Wiederbefeuchtungsleim für Briefumschläge, Briefmarken usw. eingesetzt.
2.3 Textil- und Textilpflegemittelindustrie
   Ein großes Einsatzfeld für die Stärken als Hilfsmittel und Zusatzstoff ist der Bereich Herstellung von Textilien und Textilpflegemitteln. Innerhalb der Textilindustrie sind die folgenden vier Einsatzbereiche zu unterscheiden:
   Der Einsatz der Stärke als Schlichtmittel, d.h. als Hilfsstoff zur Glättung und Stärkung des Klettverhaltens zum Schutz gegen die beim Weben angreifenden Zugkräfte sowie zur Erhöhung der Abriebfestigkeit beim Weben, Stärke als Mittel zur Textilaufrüstung vor allem nach qualitätsverschlechernden Vorbehandlungen, wie Bleichen, Färben usw., Stärke als Verdickungsmittel bei der Herstellung von Farbpasten zur Verhinderung von Farbstoffdiffusionen sowie Stärke als Zusatz zu Kettungsmitteln für Nähgarne.
2.4 Baustoffindustrie
   Der vierte Einsatzbereich ist die Verwendung der Stärken als Zusatz bei Baustoffen. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet. Weitere Einsatzbereiche sind die Beimischung zu Putz- und Mineralfasern. Bei Transportbeton werden Stärkeprodukte zur Verzögerung der Abbindung eingesetzt.
2.5 Bodenstabilisation
   Ein weiterer Markt für die Stärke bietet sich bei der Herstellung von Mitteln zur Bodenstabilisation an, die bei künstlichen Erdbewegungen zum temporären Schutz der Bodenpartikel gegenüber Wasser eingesetzt werden. Kombinationsprodukte aus der Stärke und Polymeremulsionen sind nach heutiger Kenntnis in ihrer Erosions- und verkrustungsmindernden Wirkung den bisher eingesetzten Produkten gleichzusetzen, liegen preislich aber deutlich unter diesen.
2.6 Einsatz in Pflanzenschutz- und Düngemitteln
   Ein Einsatzbereich liegt in der Verwendung der Stärke in Pflanzenschutzmitteln zur Veränderung der spezifischen Eigenschaften der Präparate. So kann die Stärke zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt werden.
2.7 Pharmaka, Medizin und Kosmetikindustrie
   Ein weiteres Einsatzgebiet besteht im Bereich der Pharmaka, Medizin und Kosmetikindustrie. In der pharmazeutischen Industrie kann die Stärke als Bindemittel für Tabletten oder zur Bindemittelverdünnung in Kapseln eingesetzt werden. Weiterhin kann die Stärke als Tablettensprengmittel dienen, da sie nach dem Schlucken Flüssigkeit absorbieren und nach kurzer Zeit soweit quellen, daß der Wirkstoff freigesetzt wird. Medizinische Gleit- und Wundpuder basieren aus qualitativen Gründen auf Stärke. Im Bereich der Kosmetik werden Stärken beispielsweise als Träger von Puderzusatzstoffen, wie Düften und Salicylsäure eingesetzt. Ein relativ großer Anwendungsbereich für die Stärke liegt bei Zahnpasta.
2.8 Stärkezusatz zu Kohlen und Briketts
   Einen Einsatzbereich bietet die Stärke als Zusatzstoff zu Kohle und Brikett. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6 %, bei kalorierter Kohle zwischen 0,1 und 0,5 %. Des weiteren gewinnen Stärken als Bindemittel an Bedeutung, da durch ihren Zusatz zu Kohle und Brikett der Ausstoß schädlicher Stoffe deutlich vermindert werden kann.
2.9 Erz- und Kohleschlammaufbereitung
   Die Stärke kann ferner bei der Erz- und Kohleschlammaufbereitung als Flockungsmittel eingesetzt werden.
2.10 Gießereihilfsstoff
   Ein weiterer Einsatzbereich besteht als Zusatz zu Gießereihilfsstoffen. Bei verschiedenen Gußverfahren werden Kerne benötigt, die aus Bindemittelversetzten Sänden hergestellt werden. Als Bindemittel wird heute überwiegend Bentonit eingesetzt, das mit modifizierten Stärken, meist Quellstärken, versetzt ist.
   Zweck des Stärkezusatzes ist die Erhöhung der Fließfestigkeit sowie die Verbesserung der Bindefestigkeit. Darüber hinaus können die Quellstärken weitere produktionstechnische Anforderungen, wie im kalten Wasser dispergierbar, rehydratisierbar, gut in Sand mischbar und hohes Wasserbindungsvermögen, aufweisen.
2.11 Einsatz in der Kautschukindustrie
   In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens, dafür wird Stärke vor der Kaltvulkanisation auf die klebrigen gummierten Flächen von Kautschukstoffen gestreut, sowie die Verbesserung der Bedruckbarkeit des Kautschuks.
2.12 Herstellung von Lederersatzstoffen
   Eine weitere Absatzmöglichkeit der modifizierten Stärken besteht bei der Herstellung von Lederersatzstoffen.
2.13 Stärke in synthetischen Polymeren
   Auf dem Kunststoffsektor zeichnen sich folgende Einsatzgebiete ab: die Einbindung von Stärkefolgeprodukten in den Verarbeitungsprozeß (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).

Die Verwendung der Stärke als reinem Füllstoff ist verglichen mit den anderen Stoffen wie Talkum nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyethylen. Hierbei werden die Stärke und das synthetische Polymer durch Koexpression im Verhältnis von 1 : 1 zu einem 'master batch' kombiniert, aus dem mit granuliertem Polyethylen unter Anwendung herkömmlicher Verfahrenstechniken diverse Produkte hergestellt werden. Durch die Einbindung von Stärke in Polyethylenfolien kann eine erhöhte Stoffdurchlässigkeit bei Hohlkörpern, eine verbesserte Wasserdampfdurchlässigkeit, ein verbessertes Antistatikverhalten, ein verbessertes Antiblockverhalten sowie eine verbesserte Bedruckbarkeit mit wäßrigen Farben erreicht werden.

Eine andere Möglichkeit ist die Anwendung der Stärke in Polyurethanschäumen. Mit der Adaption der Stärkederivate sowie durch die verfahrenstechnische Optimierung ist es möglich, die Reaktion zwischen synthetischen Polymeren und den Hydroxygruppen der Stärken gezielt zu steuern. Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurch-lässigkeit ohne Veränderung der Wasseraufnahme, Verringerung der Entflammbarkeit und der Aufrißdichte, kein Abtropfen brennbarer Teile, Halogenfreiheit und verminderte Alterung. Nachteile, die gegenwärtig noch vorhanden sind, sind verringerte Druckfestigkeit sowie eine verringerte Schlagfestigkeit.

Die Produktentwicklung beschränkt sich inzwischen nicht mehr nur auf Folien. Auch feste Kunststoffprodukte, wie Töpfe, Platten und Schalen, sind mit einem Stärkegehalt von über 50 % herzustellen. Desweiteren sind Stärke/Polymermischungen günstig zu beurteilen, da sie eine sehr viel höhere biologische Abbaubarkeit aufweisen.

Außerordentliche Bedeutung haben weiterhin auf Grund ihres extremen Wasserbindungsvermögen Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfpolymerisate zeichnen sich durch ein besseres Binde- und Rückhaltevermögen von bis zu 1000 g Wasser pro g Stärke bei hoher Viskosität aus. Die Anwendungsbereiche für diese Superabsorber haben sich in den letzten Jahren stark ausgeweitet und liegen im Hygienebereich mit Produkten wie Windeln und Unterlagen sowie im landwirtschaftlichen Sektor, z.B. bei Saatgutpillierungen.

Entscheidend für den Einsatz der neuen, gentechnisch veränderten Stärken sind zum einen die Struktur, Wassergehalt, Proteingehalt, Lipidgehalt, Fasergehalt, Asche/Phosphatgehalt, Amylose/Amylopektinverhältnis, Molmassenverteilung, Verzweigungsgrad, Korngröße und -form sowie Kristallinität, zum anderen auch die Eigenschaften, die in folgende Merkmale münden: Fließ- und Sorptionsverhalten, Verkleisterungstemperatur, Viskosität, Viskositätsstabilität in Salzlösungen, Dickungsleistung, Löslichkeit, Kleisterstruktur und -transparenz, Hitze-, Scher- und Säurestabilität, Retrogradationsneigung, Gelbildung, Gefrier/Taustabilität, Komplexbildung, Jodbindung, Filmbildung, Klebekraft, Enzymstabilität, Verdaulichkeit und Reaktivität.

Die Erzeugung modifizierter Stärken mittels gentechnischer Verfahren kann zum einen die Eigenschaften der aus der Pflanze gewonnenen Stärke dahingehend verändern, daß weitere Modifikationen mittels chemischer oder physikalischer Verfahren nicht mehr notwendig erscheinen. Zum anderen können die durch gentechnische Verfahren veränderte Stärken weiteren chemischen Modifikationen unterworfen werden, was zu weiteren Verbesserungen der Qualität für bestimmte der oben beschriebenen Einsatzgebiete führt. Diese chemischen Modifikationen sind grundsätzlich bekannt. Insbesondere handelt es sich dabei um Modifikationen durch Hitzebehandlung, Behandlung mit organischen oder anorganischen Säuren, Oxidation und Veresterungen, welche z.B. zur Entstehung von Phosphat-, Nitrat-, Sulfat-, Xanthat-, Acetat- und Citratstärken führen. Desweiteren können ein- oder mehrwertige Alkohole in Gegenwart starker Säuren zur Erzeugung von Stärkeethern eingesetzt werden, so daß Stärke-Alkylether, O-Allylether, Hydroxylalkytether, O-Carboxylmethylether, N-haltige Stärkeether, P-haltige Stärkeether), S-haltige Stärkeether, vernetzte Stärken oder Stärke-Pfropf-Polymerisate resultieren.

Eine bevorzugte Verwendung der erfindungsgemäße Stärken liegt in der Herstellung von Verpackungsmaterial und Einwegartikeln einerseits sowie als Lebensmittel oder Lebensmittelvorprodukt andererseits.

Zur Expression der erfindungsgemäßen Nucleinsäuremoleküle in sense- oder antisense-Orientierung in pflanzlichen Zellen werden diese mit regulatorischen DNA-Elementen verknüpft, die die Transkription in pflanzlichen Zellen gewährleisten. Hierzu zählen insbesondere Promotoren, Enhancer und Terminatoren. Generell kommt für die Expression jeder in pflanzlichen Zellen aktive Promotor in Frage.

Der Promotor kann dabei so gewählt sein, daß die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein. Geeignete Promotoren sind z.B. der Promotor der 35S RNA des Cauliflower Mosaic Virus und der Ubiquitin-Promotor aus Mais für eine konstitutive Expression, der Patatin-Promotor B33 (Rocha-Sosa et al., EMBO J. 8 (1989), 23-29) für eine knollenspezifische Expression oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451) oder für eine endosperm-spezifische Expression der HMG-Promotor aus Weizen, der USP-Promotor, der Phaseolinpromotor oder Promotoren von Zein-Genen aus Mais.

Ferner kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. Gielen et al., EMBO J. 8 (1989), 23-29) und sind beliebig austauschbar.

Die vorliegende Erfindung stellt Nucleinsäuremoleküle zur Verfügung, die ein Protein mit der Funktion einer Isoamylase aus Weizen codieren. Die erfindungsgemäßen Nucleinsäuremoleküle erlauben die Herstellung dieses Enzyms, dessen funktionale Identifizierung innerhalb der Stärkebiosynthese, die Herstellung gentechnisch veränderter Pflanzen, bei denen die Aktivität dieses Enzyms verändert ist und ermöglicht somit die Synthese einer Stärke mit veränderter Struktur und veränderten physikalisch-chemischen Eigenschaften in derartig modifizierten Pflanzen.

Die erfindungsgemäßen Nucleinsäuremoleküle können prinzipiell auch dazu verwendet werden, Pflanzen herzustellen, in denen die Aktivität der erfindungsgemäßen Isoamylase erhöht oder verringert ist und gleichzeitig die Aktivitäten anderer, an der Stärkesynthese beteiligter Enyzme verändert sind. Durch die Veränderung der Aktivitäten einer Isoamylase in Pflanzen kommt es zur Synthese einer in ihrer Struktur veränderten Stärke. Ferner können Nucleinsäuremoleküle, die eine Isoamylase codieren oder entsprechende antisense-Konstrukte, in Pflanzenzellen eingebracht werden, bei denen bereits die Synthese endogener Stärkesynthasen oder von Verzweigungsenzymen inhibiert ist (wie z.B. in WO 92/14827 oder Shannon und Garwood, 1984, in Whistler, BeMiller und Paschall, Starch:Chemistry and Technology, Academic Press, London, 2nd Edition: 25-86).

Soll die Inhibierung der Synthese mehrerer an der Stärkebiosynthese beteiligter Enzyme in transformierten Pflanzen erreicht werden, so können DNA-Moleküle zur Transformation verwendet werden, die gleichzeitig mehrere, die entsprechenden Enzyme codierenden Regionen in antisense-Orientierung unter der Kontrolle eines geeigneten Promotors enthalten. Hierbei kann alternativ jede Sequenz unter der Kontrolle eines eigenen Promotors stehen, oder die Sequenzen können als Fusion von einem gemeinsamen Promotor transkribiert werden bzw. unter der Kontrolle eines gemeinsamen Promoters stehen. Letztere Alternative wird in der Regel vorzuziehen sein, da in diesem Fall die Synthese der entsprechenden Proteine in etwa gleichem Maße inhibiert werden sollte. Für die Länge der einzelnen codierenden Regionen, die in einem derartigen Konstrukt verwendet werden, gilt das, was oben bereits für die Herstellung von antisense-Konstrukten ausgeführt wurde. Eine obere Grenze für die Anzahl der in einem derartigen DNA-Molekül von einem Promotor aus transkribierten antisense-Fragmente gibt es prinzipiell nicht. Das entstehende Transkript sollte aber vorzugsweise eine Länge von 10 kb und insbesondere eine Länge von 5 kb nicht überschreiten.

Codierende Regionen, die in derartigen DNA-Molekülen in Kombination mit anderen codierenden Regionen in antisense-Orientierung hinter einem geeigneten Promotor lokalisiert sind, können aus DNA-Sequenzen stammen, die für folgende Proteine codieren: Stärkekorn-gebundene (GBSS I und II) und lösliche Stärkesynthasen (SSS I und II), Verzweigungsenzyme (Isoamylasen, Pullulanasen, R-Enzyme, "Branching"-Enzyme, "Debranching"-Enzyme), Stärkephosphorylasen und Disproportionierungs-enzyme. Dies ist nur eine beispielhafte Aufzählung. Auch die Verwendung anderer DNA-Sequenzen im Rahmen einer derartigen Kombination ist denkbar.

Mit Hilfe derartiger Konstrukte ist es möglich, in Pflanzenzellen, die mit diesen transformiert wurden, die Synthese mehrerer Enzyme gleichzeitig zu inhibieren.

Weiterhin können die Konstrukte in pflanzliche Mutanten eingebracht werden, die für ein oder mehrere Gene der Stärkebiosynthese defekt sind (Shannon und Garwood, 1984, in Whistler, BeMiller und Paschall, Starch:Chemistry and Technology, Academic Press, London, 2nd Edition: 25-86). Diese Defekte können sich auf folgende Proteine beziehen: Stärkekorn-gebundene (GBSS I und II) und lösliche Stärkesynthasen (SSS I und II), Verzweigungsenzyme (BE I und II), "Debranching"-Enzyme (R-Enzyme), Disproportionierungsenzyme und Stärkephosphorylasen. Dies ist nur eine beispielhafte Aufzählung.

Mit Hilfe einer derartigen Vorgehensweise ist es weiterhin möglich, in Pflanzenzellen, die mit diesen transformiert wurden, die Synthese mehrerer Enzyme gleichzeitig zu inhibieren.

Zur Vorbereitung der Einführung fremder Gene in höhere Pflanzen stehen eine große Anzahl von Klonierungsvektoren zur Vefügung, die ein Replikationssignal für E.coli und ein Markergen zur Selektion transformierter Bakterienzellen enthalten. Beispiele für derartige Vektoren sind pBR322, pUC-Serien, M13mp-Serien, pACYC184 usw.. Die gewünschte Sequenz kann an einer passenden Restriktionsschnittstelle in den Vektor eingeführt werden. Das erhaltene Plasmid wird für die Transformation von E.coli-Zellen verwendet. Transformierte E.coli-Zellen werden in einem geeigneten Medium gezüchtet, anschließend geerntet und lysiert. Das Plasmid wird wiedergewonnen. Als Analysemethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und gewonnene DNA-Fragmente mit anderen DNA-Sequenzen verknüpft werden. Jede Plasmid-DNA-Sequenz kann in den gleichen oder anderen Plasmiden cloniert werden.

Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von Agrobacterium tumefaciens oder Agrobacterium rhizogenes als Transformationsmittel, die Fusion von Protoplasten, die Injektion, die Elektroporation von DNA, die Einbringung von DNA mittels der biolistischen Methode sowie weitere Möglichkeiten.

Bei der Injektion und Elektroporation von DNA in Pflanzenzellen werden an sich keine speziellen Anforderungen an die verwendeten Plasmide gestellt. Es können einfache Plasmide wie z.B. pUC-Derivate verwendet werden. Sollen aber aus derartig transformierten Zellen ganze Pflanzen regeneriert werden, ist in der Regel die Anwesenheit eines selektierbaren Markergens notwendig.

Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der Ti- und Ri-Plasmid T-DNA als Flankenbereich mit den einzuführenden Genen verbunden werden.

Werden für die Transformation Agrobakterien verwendet, muß die einzuführende DNA in spezielle Plasmide cloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E.coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al. Mol. Gen. Genet. 163 (1978), 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium läßt sich zur Transformation von Pflanzenzellen verwenden.

Die Verwendung von T-DNA für die Transformation von Pflanzenzellen ist intensiv untersucht und ausreichend in EP 120 516; Hoekema, In: The Binary Plant Vector System Offsetdrukkerij Kanters B.V., Alblasserdam (1985), Chapter V; Fraley et al., Crit. Rev. Plant. Sci., 4, 1-46 und An et al. EMBO J. 4 (1985), 277-287 beschrieben worden.

Für den Transfer der DNA in die Pflanzenzelle können Pflanzen-Explantate zweckmäßigerweise mit Agrobacterium tumefaciens oder Agrobacterium rhizogenes kokultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches u.a. bestimmte Zucker, Aminosäuren, Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplastentransformation sind bekannt (vgl. z.B. Willmitzer, L., 1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basel-Cambridge).

Während die Transformation dikotyler Pflanzen über Ti-Plasmid-Vektorsysteme mit Hilfe von Agrobacterium tumefaciens wohl etabliert ist, weisen neuere Arbeiten darauf hin, daß auch monokotyle Pflanzen der Transformation mittels Agrobacterium basierender Vektoren sehr wohl zugänglich sind (Chan et al., Plant Mol. Biol. 22 (1993), 491-506; Hiei et al., Plant J. 6 (1994), 271-282).

Alternative Verfahren zur Transformation von monokotylen Pflanzen bestehen mittels des biolistischen Ansatzes, der Protoplastentransformation oder der physikalisch oder chemisch induzierten DNA-Aufnahme in Protoplasten, z.B. durch Elektroporation von partiell permeabilisierten Zellen, Transfer von DNA mittels Glasfasern, Makroinjektion von DNA in Blütenstände, die Mikroinjektion von DNA in Mikrosporen oder Pro-Embryonen, die DNA-Aufnahme durch keimenden Pollen und die DNA-Aufnahme in Embryonen durch Quellung (zur Übersicht: Potrykus, Physiol. Plant (1990), 269 - 273).

Drei der oben genannten Transformationssysteme konnten in der Vergangenheit für verschiedene Getreide etabliert werden: die Elektroporation von Gewebe, die Transformation von Protoplasten und der DNA-Transfer durch Partikel-Beschuß in regenerierbare Gewebe und Zellen (zur Übersicht: Jähne et al., Euphytica 85 (1995), 35 - 44).

Die Transformation von Weizen wird in der Literatur verschiedentlich beschrieben (zur Übersicht: Maheshwari et al., Critical Reviews in Plant Science 14 (2) (1995), 149 bis 178): Hess et al. (Plant Sci. 72 (1990), 233) benutzten das Verfahren der Makroinjektion, um Pollen und Agrobakterien in unmittelbare Nähe zu bringen. Die Mobilisierung des Plasmids, das das nptII Gen als selektierbaren Marker enthielt, wurde mittels Southern Blot Analyse und NPTII Test nachgewiesen. Die Transformanten zeigten einen normalen Phänotyp und waren fertil. Die Kanamycin-Resistenz konnte in zwei aufeinanderfolgende Generationen nachgewiesen werden.

Die erste transgene, fertile Weizenpflanze, die nach Beschuß mit Mikroprojektilgebundener DNA regeneriert werden konnte, wurde von Vasil et al. (Bio/Technology 10 (1992), 667 - 674) beschrieben. Das Zielgewebe für den Beschuß war eine embryogene Kalluskultur (Typ C Kallus). Als Selektionsmarker wurde das bar Gen eingesetzt, das eine Phosphinothricin Acetyltransferase codiert und somit eine Resistenz gegen das Herbizid Phosphinothricin vermittelt. Ein weiteres System wurde von Weeks et al. (Plant Physiol. 102 (1993), 1077 - 1084), sowie Becker et al. (Plant J. 5(2) (1994), 299 - 307) beschrieben. Hier ist das Zielgewebe für die DNA-Transformation das Skutellum unreifer Embryonen, das in einer einleitenden in vitro Phase zur Induktion somatischer Embryonen angeregt wurde. Die Effizienz der Transformation liegt bei dem von Becker et al. (loc cit.) entwickelten System mit 1 transgene Pflanze pro 83 Embryonen der Sorte "Florida" deutlich höher als bei dem von Weeks et al. etablierten System mit 1 bis 2 transgenen Pflanzen pro 1000 Embryonen der Sorte "Bohwhite".

Das von Becker et al. (loc. Cit) entwickelte System bildet die Basis für die in den Beispielen beschriebenen Transformationsexperimente.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen der oben erwähnten Selektionsmarker, der den transformierten Pflanzenzellen z.B. Resistenz gegenüber einem Biozid wie Phosphinothricin oder einem Antibiotikum wie Kanamycin, G 418, Bleomycin oder Hygromycin vermittelt oder die Selektion über die An- oder Abwesenheit bestimmter Zucker oder Aminosäuren gestattet. Der individuell gewählte Marker sollte daher die Selektion transformierter Zellen gegenüber Zellen gestatten, denen die eingeführte DNA fehlt.

Die transformierten Zellen wachsen innerhalb der Pflanze in der üblichen Weise (siehe auch McCormick et al., Plant Cell Reports 5 (1986), 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften. Von den Pflanzenzellen können Samen gewonnen werden. Es sollten zwei oder mehrere Generationen angezogen werden, um sicherzustellen, daß das phänotypische Merkmal stabil beibehalten und vererbt wird. Auch sollten Samen geerntet werden, um sicherzustellen, daß der entsprechende Phänotyp oder andere Eigenarten erhalten geblieben sind.

Die nachfolgenden Beispiele sollen die Erfindung illustrieren und stellen keinerlei Beschränkung dar.

### 1. Clonierungsverfahren

Zur Clonierung in E. coli wurde der Vektor pBluescript II SK (Stratagene) verwendet.

### 2. Bakterienstämme

Für den Bluescript-Vektor und für die antisense-Konstrukte wurde der E. coli Stamm DH5α (Bethesda Research Laboratories, Gaithersburg, USA) verwendet. Für die in vivo Excision wurde der E. coli-Stamm XL1-Blue verwendet.

### 3. Transformation unreifer Weizenembryonen

### Medien

| | | |
|---|---|---|
| MS | 100 ml/l Makrosalz 1 ml/l Mikrosalz 2 ml/l Fe/NaEDTA 30 g/l Saccharose | (D.Becker und H. Lörz, Plant Tissue Culture Manual (1996), B 12:1-20) |
| | | |
| #30 | MS + 2,4-D (2 mg/l) | |
| | | |
| #31 | MS + 2,4-D (2 mg/l) + Phosphinothricin (PPT, aktive Komponente des Herbizids BASTA (2 mg/l)) | |
| | | |
| #32 | MS + 2,4-D (0,1 mg/l) + PPT (2 mg/l) | |
| | | |
| #39 | MS + 2,4-D (2 mg/ml) + je 0,5 N Mannit/Sorbit | |

Die angegebenen Medien wurden auf den pH-Wert 5,6 mit KOH eingestellt und mit 0,3 % Gelrite verfestigt.

Die Methode zur Transformation unreifer Embryonen aus Weizen wurde von Becker und Lörz (D. Becker und H. Lörz, Plant Tissue Culture Manual (1996), B12: 1 bis 20) entwickelt und optimiert.

In den nachfolgend beschriebenen Experimenten wurde sich an das von Becker und Lörz (loc. Cit) ausgearbeitete Protokoll gehalten.

Zur Transformation werden Ähren mit Karyopsen der Entwicklungstufe 12 bis 14 Tage nach Anthesis geerntet und oberflächensterilisiert. Die isolierten Skutella werden mit der dem Medium zugewandten Embryoachse auf Induktionsmedium # 30 plattiert.

Nach 2 bis 4 tägiger Vorkultur (26°C, dunkel) werden die Explantate auf Medium
# 39 zur osmotischen Vorkultur (2 bis 4 h, 26°C, dunkel) umgesetzt.

Zur biolistischen Transformation werden pro Schuß ca. 29 µg Goldpartikel, auf die zuvor wenige µg der Ziel-DNA gefällt wurde, eingesetzt. Da es sich bei den durchgeführten Experimenten um Co-Transformanten handelt, wird die Ziel-DNA in einem Verhältnis von 1:1, bestehend aus dem Zielgen und einem Resistenzmarkergen (bar-Gen) dem Fällungsansatz zugegeben.

### 4. DIG-Markierung von DNA-Fragmenten

Die Markierung von DNA-Fragmenten, die als Screeningsonden eingesetzt wurden, erfolgte über eine spezifische PCR unter Einbau von DIG-markiertem dUTP (Boehringer Mannheim, Deutschland).

In den Beispielen verwendete Medien und Lösungen:

| | |
|---|---|
| 20 x SSC | 175,3 g NaCl |
| | 88,2 g Natrium-Citrat |
| | ad 1000 ml mit ddH₂O |
| | pH 7,0 mit 10 N NaOH |

Bei der DSMZ in Braunschweig, Bundesrepublik Deutschland, erfolgte die Hinterlegung des Plasmids pTaSU 8A gemäß Budapester Vertrag unter der Nr. DSM 12795 sowie des Plasmids pTaSU 19 unter der Nr. DSM 12796.

### Beispiel 1: Identifizierung, Isolierung und Charakterisierung einer cDNA, die eine Isoamylase (sugary-homolog) aus Weizen (Triticum aestivum L., cv Florida) codiert

Zur Identifizierung einer cDNA, die eine Isoform einer Isoamylase (sugary) aus Weizen codiert, wurde die Strategie des heterologen Screening verfolgt. Hierfür wurde eine cDNA-Bank aus Weizen mit einer sugary-Sonde aus Mais durchmustert.

Die Isolierung der Sonde (sugary-Sonde) aus einer Mais cDNA Bank erfolgte mittels spezifischer Primer durch PCR-Amplifikation. Die Clonierung der Mais cDNA Bank erfolgte aus poly(A) + RNA aus einem Gemisch gleicher Anteile 13,17,19,20,22,25 und 28 Tage (DAP) alter Karyopsen in einem Lambda Zap II Vektor analog den Angaben des Herstellers (Lambda ZAP II-cDNA Synthesis Kit Stratagene GmbH, Heidelberg, Deutschland). Bei allen verwendeten Karyopsen, außer bei den 13 Tage alten Körnern, war vor der RNA-Isolierung der Embryo entfernt worden.

Die Amplifizierung des DNA-Fragmentes, das als Sonde zur Durchmusterung der Weizen cDNA Bank eingesetzt wurde, erfolgte mit den folgenden Primern:

Als Template für die PCR-Reaktion wurden 2 µl der amplifizierten Mais cDNA Bank eingesetzt. Die PCR-Reaktion enthielt des weiteren 1,5-3mM MgCl₂, 20mM Tris-HCl (pH 8,4), 50mM KCI, 0.8mM dNTP Mix, 1µM Primer su1 p-1 a, 1µM Primer su1 p-2 und 2.5 Units Taq Polymerase (rekombinant, Life Technologies). Die Amplifizierung wurde mit einem Trioblock der Firma Biometra nach dem Schema: 4'(min)/ 95°C;1'/ 95°C; 45"(sek)/ 58°C; 1'15"/ 72°C; 30 Cyclen 5'/ 72°C. Die amplifizierte DNA Bande von ca. 990 bp wurde in einem Agarosegel aufgetrennt und ausgeschnitten. Von diesem Fragment wurde nach dem gleichen Schema wie oben beschrieben eine zweite Amplifizierung durchgeführt. Das aus dieser zweiten Amplifizierung erhaltene 990 bp Fragment wurde mit dem Restriktionsenzym *BAM* HI in ein 220 bp und ein 770 bp Fragment zerschnitten. Nach nochmaliger Auftrennung des sugary-Fragmentes in einem Agarosegel, Ausschneiden der Bande und Isolierung des Fragmentes erfolgte die DIG-Markierung der Sonde. Zur 'random prime' Markierung mit Digoxygenin wurden 500 ng sugary Fragment eingesetzt. Zu dem zu markierenden Fragment wurden 10 µl Random Primer gegeben und die Reaktion wurde 5' bei 95-100°C erhitzt. Nach dem Erwärmen wurden 0,1 mM dATP, 0,1 mM dGTP, 0,1 mM dCTP und 0,065mM dTTP und 0,035 mM Digoxygenin-11-dUTP (Boehringer Mannheim), sowie Klenow Puffer (Standard) und 1 Unit Klenow Polymerase zugegeben. Die Reaktion wurde bei RT (Raumtemperatur) über Nacht durchgeführt. Zur Kontrolle der Markierung wurde ein Dot-Test analog den Angaben des Herstellers ('The DIG System User's Guide for Filter Hybridization' der Firma Boehringer, Mannheim, Deutschland) durchgeführt.

Die Synthese der Weizen cDNA Bank erfolgte aus poly(A) + RNA von ca.21 Tage ('starchy'-Endosperm) alten Karyopsen in einen Lamda Zap II Vektor entsprechend den Angaben des Herstellers (Lambda ZAP II-cDNA Synthesis Kit, Stratagene GmbH, Heidelberg). Nach Titerbestimmung der cDNA-Bank konnte ein Primärtiter von 1,26 x 10⁶ pfu/ml ermittelt werden.

Zum Durchmustern der Weizen cDNA Bank wurden ca. 350.000 Phagen ausplattiert. Ausplattieren der Phagen und Abziehen der Platten erfolgte nach Standardprotokollen. Die Prähybridisierung und Hybridisierung der Filter wurde in 5X SSC, 3% Blocking (Boehringer, Mannheim), 0,2% SDS, 0,1% Natrium Laurylsarcosin und 50 µg/ml Heringssperma DNA bei 55°C durchgeführt. Der Hybridisierungslösung wurde 1ng/ml der markierten sugary Sonde zugesetzt und die Hybridisierung über Nacht inkubiert. Gewaschen wurden die Filter für 2X5' in 2X SSC, 1% SDS bei RT; 2X 10' in 1X SSC, 0,5% SDS bei 55°C; 2x 10' in 0,5X SSC, 0,2% SDS bei 55°C. Positive Clone wurden durch weitere Screening Runden vereinzelt. Über *in vivo* Excision wurden vereinzelte Clone als pBluescript SK Phagemide erhalten (Durchführung analog den Angaben des Herstellers; Stratagene, Heidelberg, Deutschland).

Nach Analyse der Clone über Minipräparierung und Restringierung der Plasmid-DNA wurde der Clon pTaSU -19 bei der DSMZ-Deutschen Sammlung für Mikroorganismen und Zellkulturen GmbH unter der Nummer DSM 12796 hinterlegt und weiter analysiert.

### Beispiel 2: Sequenzanalyse der cDNA-Insertionen des Plasmids pTaSU19

Aus dem Clon pTaSU19 wurde die Plasmid-DNA isoliert und die Sequenz der cDNA-Insertionen mittels der Didesoxynukleotidmethode (Sanger et al., Proc. Natl. Acad. Sci. USA 74 (1977), 5463-5467) bestimmt.

Die Insertion des Clons TaSU-19 ist 2997 bp lang und stellt eine partielle cDNA dar. Die Nucleotidsequenz ist unter Seq ID No. 1 angegeben.
Eine Vergleich mit bereits publizierten Sequenzen zeigte, daß die unter Seq ID No. 1 dargestellte Sequenz eine codierende Region umfaßt, die Homologien zu Isoamylasen aus anderen Organismen aufweist.
Die Analyse der Sequenz zeigt auch, daß sich in der cDNA Sequenz zwei Introns in den Positionen 297-396 (Intron 1) und 1618-2144 (Intron 2) befinden.
Werden diese Introns entfernt, läßt sich eine Proteinsequenz ableiten, die Homolgien zu den Proteinsequenzen von Isoamylasen anderer Organismen aufweist. Die zu den codierenden Bereichen der Seq ID No. 1 korrespondierende Aminosäure-Sequenz ist unter Seq ID No. 3 dargestellt.

### Beispiel 3 Herstellung des Pflanzentransformationsvektors pTa-alpha-SU19

Für die Expression einer zur TaSU19-cDNA korrespondierenden antisense-RNA wurden auf der Grundlage des Basisplasmids pUC19 der Pflanzentransformationsvektoren pTa-alpha-SU19 konstruiert, indem die cDNA-Insertion des Plasmids pTa-alpha-SU19 in antisense-Orientierung mit dem 3' Ende des Ubiquitin-Promotors verbunden wurden. Dieser Promotor besteht aus dem ersten untranslatierten Exon und dem ersten Intron des *ubiquitin 1* Gens aus Mais (Christensen A.H. et al., Plant Molecular Biology 18 (1992), 675-689).Teile des Polylinkers und der NOS-Terminator stammen aus dem Plasmid pAct1.cas (CAMBIA, TG 0063; Cambia, GPO Box 3200, Canberra ACT 2601, Australia). Vektorkonstrukte mit diesem Terminator und Konstrukten, die auf pAct1.cas basieren, sind in MCElroy et al. (Molecular Breeding 1 (1995), 27-37) beschrieben. Der so entstandene Vektor wurde pUbi.cas genannt.

Die Klonierung des Vektors erfolgt durch Restriktion eines 2kb Fragmentes aus dem Klon Ta-SU19 mit dem Restriktionsenzym *Xba I.* Das Fragment wurde an den Enden mittels einer Klenow Reaktion aufgefüllt und anschließend in die *Sma* / Klonierungsstelle des Expressionsvektors pUbi.cas ligiert.

Der entstandene Expressionsvektor wird als Ta-alpha-SU 19 bezeichnet und wird wie oben beschrieben zur Transformation von Weizen verwendet.

### Beispiel 4: Isolierung und Charakterisierung einer weiteren cDNA, die für eine Isoamylase (Sugary1-homolog) aus Weizen (Triticum aestivum L., cv Florida) kodiert

Eine cDNA-Bank aus Weizen wurde mit einer Sugary-Sonde durchmustert, die einen Teil des Klons pTaSU19, und zwar Position 489-1041 aus Seq.ID No. 1, repräsentiert.

Die Herstellung der weizenspezifischen Digoxygenin-markierten Sugary-Sonde, die zur Durchmusterung der cDNA-Bank eingesetzt wurde, erfolgte mittels PCR-Amplifizierung. Die in dieser Reaktion eingesetzten Primer waren: und

Als Template wurde 1 ng des Plasmids pTaSU19 in die Reaktion eingesetzt. Die PCR-Reaktion enthielt außerdem je 300 nM der Primer SUS01 und SUS02, je 100 µM der Nukleotide dATP, dGTP, dCTP, 65 µM dTTP, 35 µM Digoxygenin-11-dUTP (Boehringer Mannheim), 1,5 mM MgCl₂, sowie 2,5 U (Units) Taq Polymerase und 10 µl 10-fach konzentrierten Taq Polymerase Reaktionspuffer (beides Life Technologies). Das Endvolumen der Reaktion betrug 100 µl.
Die Amplifizierung erfolgte auf einem PCR-Gerät (TRIO®Thermoblock, Biometra) unter folgendem Temperaturprogramm: 3' (min) bei 95°C (1-mal); 45" (sek) bei 95°C - 45" bei 55°C - 2' bei 72°C (30 Zyklen); 5' bei 72°C (1-mal). Es resultierte ein DNA-Fragment von 553 bp Länge. Der Einbau von Dogoxygenin-11-dUTP in das PCR-Produkt zeigte sich anhand der geringeren Mobilität im Agarosegel im Vergleich zu dem Produkt einer Kontrollreaktion ohne Digoxygenin-11-dUTP.

Die Karyopsen-spezifische cDNA-Bank aus Weizen aus Beispiel 1 wurde mit der erhaltenen Digoxygenin-markierten Sonde durchmustert.

Der Hybridisierungsschritt erfolgte über Nacht in 5x SSC, 0.2%SDS, 0.1 % Na-Laurylsarcosin und 50 µg/ml Heringssperma DNA bei 68°C in Gegenwart von 1 ng/ml der Digoxygenin-markierten Sonde. Nach der Hybridisierung wurden die Filter in folgender Weise gewaschen: 2x5' in 2x SSC, 1 % SDS bei RT; 2x10' in 1 x SSC, 0.5% SDS bei 68°C; 2x 10' in 0,5x SSC, 0,2% SDS bei 68°C. Positive Klone wurden durch mindestens zwei weitere Screening-Runden vereinzelt. Über *in vivo* Excision wurden aus den Phagenklonen pBluescript SK Plasmide erhalten (Protokolle nach Angaben des Herstellers; Stratagene, Heidelberg, Deutschland). Nach Restriktionsanalayse er erhaltenen Klone wurde der Klon pTaSU8A bei der Deutschen Sammlung für Mikroorganismen und Zellkulturen unter der Nummer DSM 12795 hinterlegt und weiter untersucht.

### Beispiel 5: Sequenzanalyse des cDNA-Inserts im Plasmid pTaSU8A

Die Nukleotidsequenz des cDNA-Inserts im Plasmid pTaSU8A wurde mittels der Didesoxynukleotidmethode bestimmt (Seq.ID No.6).

Die Insertion des Klons pTaSU8A ist 2437 bp lang und stellt eine partielle cDNA dar. Ein Vergleich zu bereits publizierten Sequenzen zeigt, daß die unter Seq.ID No.6 dargestellte Sequenz eine codierende Region umfaßt, die Homologien zu Isoamylasen aus weiteren Organismen aufweist. Gleichfalls zeigt die vom kodierenden Bereich des Klons pTaSU8A abgeleitete Proteinsequenz, dargestellt in Seq.ID No. 7, Homologien zu den Proteinsequenzen von Isoamylasen anderer Organismen. Vergleicht man die Sequenzen der Klone pTaSU19 (Seq.ID No. 1) und pTaSU8A (Seq.ID No. 6), so ergibt sich eine Ähnlichkeit von 96,8%. Die meisten Sequenzunterschiede liegen im 3'-untranslatierten Bereich der cDNAs. Die übrigen Sequenzunterschiede im kodierenden Bereich bedingen verschiedene Aminsäuren an insgesamt zwölf Positionen der abgeleiteten Proteinsequenzen (Seq.ID No. 3 und 7). Die in pTaSU19 und pTaSU8A enthaltenen cDNAs sind nicht identisch und kodieren für Isoformen der Isoamylase aus Weizen.

### Beispiel 6: Herstellung des Pflanzentransformationsvektors pTa-alpha-SU8A

Für die Expression einer zur TaSU8A-cDNA korrrespondierenden antisense-RNA wurde auf der Grundlage des Basisplasmids pUC19 der Pflanzentransformationsvektor pTa-alpha-SU8A-konstruiert, indem ein durch PCR-Amplifizierung hergestellter Teil der TaSU8A-cDNA in antisense-Orientierung mit dem 3'-Ende des Ubiquitin-Promotors verbunden wurde. Dieser Promotor besteht aus dem ersten untranslatierten Exon und dem ersten Intron des *ubiquitin* / Gens aus Mais (Christensen A.H. et al., Plant Mol.Biol. 18 (1992), 675-689). Teile des Polylinkers und der NOS-Terminator stammen aus dem Plasmid pAct1.cas (CAMBIA, TG 0063; Cambia , GPO Box 3200, Canberra ACT 2601, Australia).
Vektorkonstrukte mit diesem Terminator sowie Konstrukte, die auf pAct1.cas basieren, sind von McElroy et al. (Molecular Breeding 1 (1995), 27-37) beschrieben worden. Der Vektor mit Ubiquitin-Promotor, Polylinker und NOS-Terminator auf der Basis von pUC19 wurde pUbi.cas bezeichnet.

Für die Klonierung von pTa-alpha-SU8A wurde ein ca. 2,2 kb großer Teil der TaSU8A-cDNA, und zwar Position 140-2304 aus Seq.ID No.6, mittels PCR amplifiziert.

Die in dieser Reaktion eingesetzten Primer waren: und

Als Template wurde 1 ng des Plasmids pTaSU8A in die Reaktion eingesetzt. Die PCR-Reaktion enthielt außerdem: Je 300 nM der Primer SUEX3 und SUEX4, je 200 µM der Nukleotide dATP, dGTP, dCTP und dTTP, 1,6 mM MgCl₂, 60 mM Tris-SO₄ (pH 9,1) 18 mM (NH₄)₂SO₄ sowie 1 µl Elongase® Enzym Mix (Taq Polymerase und DNA Polymerase Gemisch, Life Technologies). Das Endvolumen der Reaktion betrug 50 µl. Die Amplifizierung erfolgte auf einem PCR-Gerät (TRIO® Thermoblock, Biometra) unter folgendem Temperaturprogramm: 1' (min) bei 94°C (1-mal); 30" (sek) bei 95°C - 30" bei 55°C - 2'30" bei 68°C (30 Zyklen); 10' bei 68 ° C (1-mal). In der Reaktion entstand ein DNA-Fragment von 2205 bp Länge.

Das 2,2 kb-Produkt wurde mit K*pn*l und S*al*I restringiert und in den mit K*pn*l und S*al*I vorgeschnittenen Expressionsvektor pUbi.cas ligiert. Der so entstandene Pflanzentransformationsvektor wurde pTa-alpha-SU8A genannt und wie oben beschrieben zur Transformation von Weizen verwendet.

## Patentansprüche

1. Nucleinsäuremoleküle, codierend ein Protein mit der Funktion einer Isoamylase aus Weizen, ausgewählt aus der Gruppe bestehend aus
(a) einem Nucleinsäuremolekül, das ein Protein codiert, das die unter SEQ ID NO: 3 angegebene Aminosäuresequenz umfasst;
(b) einem Nucleinsäuremolekül, das die unter SEQ ID NO: 2 dargestellte Nucleotidsequenz umfasst oder eine hierzu korrespondierende Ribonucleotidsequenz;
(c) einem Nucleinsäuremolekül, das mit einem der unter (a) oder (b) genannten Nucleinsäuremoleküle hybridisiert oder komplementär dazu ist; und
(d) einem Nucleinsäuremolekül, dessen Nucleotidsequenz aufgrund der Degeneration des genetischen Codes von der Sequenz eines unter (a), (b) oder (c) genannten Nucleinsäuremoleküls abweicht,
wobei das unter (a), (c) und (d) genannte Nucleinsäuremolekül eine Sequenzidentität von über 90% mit SEQ ID NO: 2 aufweist.

2. Nucleinsäuremolekül nach Anspruch 1, **dadurch gekennzeichnet, dass** es ein DNA-Molekül ist.

3. DNA-Molekül nach Anspruch 2, **dadurch gekennzeichnet, dass** es ein cDNA-Molekül ist.

4. Nucleinsäuremolekül nach einem oder mehreren der Ansprüche 1 bis 3, das regulatorische Elemente enthält.

5. Nucleinsäuremolekül nach Anspruch 1, **dadurch gekennzeichnet, dass** es ein RNA-Molekül ist.

6. Nucleinsäuremolekül, das spezifisch mit einem Nucleinsäuremolekül nach einem der Ansprüche 1 bis 5 hybridisiert und mit SEQ ID NO: 2 eine Sequenzidentität von über 90% aufweist.

7. Nucleinsäuremolekül nach Anspruch 6, das ein Oligonucleotid mit einer Länge von mindestens 15 Nucleotiden ist.

8. Vektor, enthaltend ein DNA-Molekül nach einem der Ansprüche 1 bis 5.

9. Vektor nach Anspruch 8, worin besagtes Nucleinsäuremolekül in sense-Orientierung mit regulatorischen Elementen verknüpft ist, die die Transkription und Synthese einer translatierbaren RNA in pro- oder eukaryontischen Zellen gewährleisten.

10. Vektor nach Anspruch 8, worin besagtes Nucleinsäuremolekül in sense-Orientierung mit regulatorischen Elementen verknüpft ist, die die Synthese einer nicht-translatierbaren RNA in pro- oder eukaryontischen Zellen gewährleisten.

11. Vektor nach Anspruch 8, worin besagtes Nucleinsäuremolekül in antisense-Orientierung mit regulatorischen Elementen verknüpft ist, die die Synthese einer nicht-translatierbaren RNA in pro- oder eukaryontischen Zellen gewährleisten.

12. Wirtszelle, die mit einem Nucleinsäuremolekül nach einem oder mehreren der Ansprüche 1 bis 5 oder einem Vektor nach einem oder mehreren der Ansprüche 8 bis 11 transformiert ist oder die von einer solchen Zelle abstammt.

13. Protein codiert durch ein Nucleinsäuremolekül nach einem oder mehreren der Ansprüche 1 bis 4.

14. Verfahren zur Herstellung eines Proteins nach Anspruch 13, worin eine Wirtszelle nach Anspruch 12 unter Bedingungen kultiviert wird, die die Synthese des besagten Proteins erlauben und besagtes Protein aus den kultivierten Zellen und/oder dem Kulturmedium isoliert wird.

15. Verfahren zur Herstellung einer transgenen Pflanzenzelle, worin
(a) ein Nucleinsäuremolekül nach einem oder mehreren der Ansprüche 1 bis 5 oder
(b) ein Vektor nach einem oder mehreren der Ansprüche 8 bis 11 in das Genom einer pflanzlichen Zelle integriert wird.

16. Transgene Pflanzenzelle, die mit einem Nucleinsäuremolekül nach einem oder mehreren der Ansprüche 1 bis 4 oder einem Vektor nach einem oder mehreren der Ansprüche 8 bis 11 transformiert wurde oder die von einer solchen Zelle abstammt.

17. Verfahren zur Herstellung einer transgenen Pflanze, worin
a1) ein Nucleinsäuremolekül nach einem oder mehreren der Ansprüche 1 bis 5; oder
a2) ein Vektor nach einem oder mehreren der Ansprüche 8 bis 11 in das Genom einer pflanzlichen Zelle integriert wird; und
b) eine vollständige Pflanze aus besagter Pflanzenzelle regeneriert wird.

18. Pflanze, enthaltend eine Pflanzenzelle nach Anspruch 16.

19. Pflanze nach Anspruch 18, die eine Nutzpflanze ist.

20. Pflanze nach Anspruch 19, die eine stärkespeichernde Pflanze ist.

21. Pflanze nach Anspruch 20, die eine monokotyle Pflanze oder Mais ist.

22. Pflanze nach Anspruch 21, die eine Gersten, Roggen oder Weizenpflanze ist.

23. Vermehrungsmaterial einer Pflanze nach einem oder mehreren der Ansprüche 18 bis 22, enthaltend eine Pflanzenzelle nach Anspruch 16.

24. Verwendung einer Pflanzenzelle nach Anspruch 16, einer Pflanze nach einem oder mehreren der Ansprüche 18 bis 22 oder von Vermehrungsmaterial nach Anspruch 23 zur Herstellung von Stärke.

## Claims

1. Nucleic acid molecules encoding a protein with the function of a wheat isoamylase, selected from the group consisting of
(a) a nucleic acid molecule encoding a protein comprising the amino acid sequence shown under SEQ ID NO:3,
(b) a nucleic acid molecule comprising the nucleotide sequence shown under SEQ ID NO:2 or a ribonucleotide sequence corresponding hereto,
(c) a nucleic acid molecule which hybridizes with a nucleic acid molecule mentioned under (a) or (b) or is complementary thereto, and
(d) a nucleic acid molecule the nucleotide sequence of which deviates from the sequence of a nucleic acid molecule mentioned under (a), (b) or (c) owing to the degeneracy of the genetic code,
wherein the nucleic acid molecule mentioned under (a), (c) and (d) has a sequence identity of more than 90% with SEQ ID NO:2.

2. The nucleic acid molecule of claim 1, which is a DNA molecule.

3. The DNA molecule of claim 2, which is a cDNA molecule.

4. The nucleic acid molecule of any one of claims 1 to 3, which contains regulatory elements.

5. The nucleic acid molecule of claim 1, which is an RNA molecule.

6. A nucleic acid molecule which specifically hybridizes with a nucleic acid molecule of any one of claims 1 to 5 and which has a sequence identity of more than 90% with SEQ ID NO:2.

7. The nucleic acid molecule of claim 6, which is an oligonucleotide with a length of at least 15 nucleotides.

8. A vector containing a DNA molecule of any one of claims 1 to 5.

9. The vector of claim 8, wherein said nucleic acid molecule is linked in sense orientation to regulatory elements which ensure transcription and synthesis of a translatable RNA in pro- or eukaryotic cells.

10. The vector of claim 8, wherein said nucleic acid molecule is linked in sense orientation to regulatory elements which ensure the synthesis of an untranslatable RNA in pro- or eukaryotic cells.

11. The vector of claim 8, wherein said nucleic acid molecule is linked in antisense orientation to regulatory elements which ensure the synthesis of an untranslatable RNA in pro- or eukaryotic cells.

12. A host cell which is transformed with a nucleic acid molecule of any one of claims 1 to 5 or a vector of any one of claims 8 to 11 or which is derived from such a cell.

13. A protein encoded by a nucleic acid molecule of any one of claims 1 to 4.

14. A process for the preparation of a protein of claim 13, wherein a host cell of claim 12 is cultured under conditions which permit said protein to be synthesized and said protein is isolated from the cultured cells and/or the culture medium.

15. A process for generating a transgenic plant cell, wherein
(a) a nucleic acid molecule of any one of claims 1 to 5, or
(b) a vector of any one of claims 8 to 11 is integrated into the genome of a plant cell.

16. A transgenic plant cell which has been transformed with a nucleic acid molecule of any one of claims 1 to 4 or a vector of any one of claims 8 to 11 or which is derived from such a cell.

17. A process for generating a transgenic plant, wherein
(a1) a nucleic acid molecule of any one of claims 1 to 5; or
(a2) a vector of any one of claims 8 to 11 is integrated into the genome of a plant cell; and
(b) an intact plant is regenerated from said plant cell.

18. A plant containing a plant cell of claim 16.

19. The plant of claim 18, which is a useful plant.

20. The plant of claim 19, which is a starch-storing plant.

21. The plant of claim 20, which is a monocotyledonous plant or maize.

22. The plant of claim 21, which is a barley, rye or wheat plant.

23. Propagation material of a plant of any one of claims 18 to 22, containing a plant cell of claim 16.

24. Use of a plant cell of claim 16, of a plant of any one of claims 18 to 22 or of propagation material of claim 23 for the production of starch.

## Revendications

1. Molécule d'acide nucléique codant pour une protéine avec la fonction d'une isoamylase de blé, prise dans le groupe comprenant
(a) une molécule d'acide nucléique, qui code pour une protéine qui comprend la séquence en acides aminés indiquée à la SEQ ID NO: 3 ;
(b) une molécule d'acide nucléique qui comprend la séquence nucléotidique représentée à la SEQ ID NO: 2 ou une séquence ribonucléotidique correspondante ;
(c) une molécule d'acide nucléique qui s'hybride avec une des molécules d'acides nucléiques citées au point (a) ou (b) ou est complémentaire de celles-ci ; et
(d) une molécule d'acide nucléique dont la séquence nucléotidique diffère de la séquence de l'une des molécules d'acide nucléique citées aux points (a), (b) ou (c) en raison de la dégénérescence du code génétique,
la molécule d'acide nucléique citée aux points (a), (c) et (d) présente une identité avec la séquence SEQ ID NO: 2 supérieure à 90 %.

2. Molécule d'acide nucléique selon la revendication 1, **caractérisée en ce qu'**elle est une molécule d'ADN.

3. Molécule d'ADN selon la revendication 2, **caractérisée en ce qu'**elle est une molécule d'ADNc.

4. Molécule d'acide nucléique selon une ou plusieurs des revendications 1 à 3, qui comprend des éléments régulateurs.

5. Molécule d'acide nucléique selon la revendication 1, **caractérisée en ce qu'**elle est une molécule d'ARN.

6. Molécule d'acide nucléique qui s'hybride spécifiquement avec une molécule d'acide nucléique selon l'une quelconque des revendications 1 à 5 et qui présente une identité de séquence avec la SEQ ID NO: 2 supérieure à 90 %.

7. Molécule d'acide nucléique selon la revendication 6, qui est un oligonucléotide long d'au moins 15 nucléotides.

8. Vecteur renfermant une molécule d'ADN selon l'une des revendications 1 à 5.

9. Vecteur selon la revendication 8, où ladite molécule d'acide nucléique est reliée aux éléments de régulation en orientation sens, qui garantissent la transcription et la synthèse d'un ARN traduisible dans les cellules procaryotes ou eucaryotes.

10. Vecteur selon la revendication 8, où ladite molécule d'acide nucléique est reliée aux éléments de régulation en orientation sens, qui garantissent la synthèse d'un ARN non-traduisible dans les cellules procaryotes ou eucaryotes.

11. Vecteur selon la revendication 8, où ladite molécule d'acide nucléique est reliée aux éléments de régulation en orientation antisens, qui garantissent la synthèse d'un ARN non-traduisible dans les cellules procaryotes ou eucaryotes.

12. Cellule-hôte qui est transformée par une molécule d'acide nucléique selon une ou plusieurs des revendications 1 à 5 ou un vecteur selon une ou plusieurs des revendications 8 à 11 ou qui provient d'une telle cellule.

13. Protéine codée par une molécule d'acide nucléique selon une ou plusieurs des revendications 1 à 4.

14. Procédé pour la préparation d'une protéine selon la revendication 13, où l'on cultive une cellule-hôte selon la revendication 12 dans des conditions qui permettent la synthèse de ladite protéine et l'isolement de ladite protéine à partir de cellules cultivées et/ou de milieu de culture.

15. Procédé pour la préparation d'une cellule végétale transgénique dans lequel
(a) une molécule d'acide nucléique selon une ou plusieurs des revendications 1 à 5, ou
(b) un vecteur selon une ou plusieurs des revendications 8 à 11 est intégré dans le génome d'une cellule végétale.

16. Cellule végétale transgénique qui est transformée à l'aide d'une molécule d'acide nucléique selon une ou plusieurs des revendications 1 à 4 ou à l'aide d'un vecteur selon une ou plusieurs des revendications 8 à 11 ou qui provient d'une telle cellule.

17. Procédé pour la préparation d'une plante transgénique dans lequel
(a1) une molécule d'acide nucléique selon une ou plusieurs des revendications 1 à 5, ou
(a2) un vecteur selon une ou plusieurs des revendications 8 à 11 est intégré dans le génome d'une cellule végétale ; et
(b) une plante entière est régénérée à partir de ladite cellule végétale.

18. Plante renfermant une cellule végétale selon la revendication 16.

19. Plante selon la revendication 18, qui est une plante utile.

20. Plante selon la revendication 19, qui est une plante de stockage d'amidon.

21. Plante selon la revendication 20, qui est une plante monocotylédone ou le mais.

22. Plante selon la revendication 21, qui est une plante d'orge, de seigle ou de blé.

23. Matière de reproduction d'une plante selon une ou plusieurs des revendications 18 à 22, renfermant une cellule végétale selon la revendication 16.

24. Utilisation d'une cellule végétale selon la revendication 16, d'une plante selon une ou plusieurs des revendications 18 à 22 ou de la matière de reproduction selon la revendication 23 pour la préparation d'amidon.


### SEQUENZPROTOKOLL

<110> Hoechst Schering AgrEvo GmbH
<120> Nucleinsäuremoleküle codierend Enzyme aus Weizen, die an der Stärkesynthese beteiligt sind
<150> DE 198 20 608.9
<151> 1998-05-08
<160> 11

<210> 1
<211> 2997
<212> DNA
<213> Triticum aestivum L. cv. Florida
<220>
<221> CDS
<222> (3) ... (296); (397) ... (1617); (2145) ... (2960)

<210> 2
<211> 2295
<212> DNA
<213> Triticum aestivum L. cv. Florida
<220>
<223> cDNA

<400> 2

<210> 3
<211> 764
<212> PRT
<213> Triticum aestivum L. cv. Florida

<400> 3
   Ser Gly Pro Ala Pro Arg Leu Arg Arg Trp Arg Pro Asn Ala Thr Ala
    1               5                  10                  15
   Gly Lys Gly Val Gly Glu Val Cys Ala Ala Val Val Glu Ala Ala Thr
               20                  25                  30
   Lys Val Glu Asp Glu Gly Glu Glu Asp Glu Pro Val Ala Glu Asp Arg
           35                  40                  45
   Tyr Ala Leu Gly Gly Ala Cys Arg Val Leu Ala Gly Met Pro Ala Pro
       50                  55                  60
   Leu Gly Ala Thr Ala Leu Ala Gly Gly Val Asn Phe Ala Val Tyr Ser
   65                  70                  75                  80
   Gly Gly Ala Thr Ala Ala Ala Leu Cys Leu Phe Thr Pro Glu Asp Leu
                   85                  90                  95
   Lys Ala Asp Arg Val Thr Glu Glu Val Pro Leu Asp Pro Leu Met Asn
               100                 105                 110
   Arg Thr Gly Asn Val Trp His Val Phe Ile Glu Gly Glu Leu His Asn
           115                 120                 125
   Met Leu Tyr Gly Tyr Arg Phe Asp Gly Thr Phe Ala Pro His Cys Gly
   130 135 140
   His Tyr Leu Asp Val Ser Asn Val Val Val Asp Pro Tyr Ala Lys Ala
   145 150 155 160
   Val Ile Ser Arg Gly Glu Tyr Gly Val Pro Ala Arg Gly Asn Asn Cys
   165 170 175
   Trp Pro Gln Met Ala Gly Met Ile Pro Leu Pro Tyr Ser Thr Phe Asp
   180 185 190
   Trp Glu Gly Asp Leu Pro Leu Arg Tyr Pro Gln Lys Asp Leu Val Ile
   195 200 205
   Tyr Glu Met His Leu Arg Gly Phe Thr Lys His Asp Ser Ser Asn Val
   210 215 220
   Glu His Pro Gly Thr Phe Ile Gly Ala Val Ser Lys Leu Asp Tyr Leu
   225 230 235 240
   Lys Glu Leu Gly Val Asn Cys Ile Glu Leu Met Pro Cys His Glu Phe
   245 250 255
   Asn Glu Leu Glu Tyr Ser Thr Ser Ser Ser Lys Met Asn Phe Trp Gly
   260 265 270
   Tyr Ser Thr Ile Asn Phe Phe Ser Pro Met Thr Arg Tyr Thr Ser Gly
   275 280 285
   Gly Ile Lys Asn Cys Gly Arg Asp Ala Ile Asn Glu Phe Lys Thr Phe
   290 295 300
   Val Arg Glu Ala His Lys Arg Gly Ile Glu Val Ile Leu Asp Val Val
   305 310 315 320
   Phe Asn His Thr Ala Glu Gly Asn Glu Asn Gly Pro Ile Leu Ser Phe
   325 330 335
   Lys Gly Val Asp Asn Thr Thr Tyr Tyr Met Leu Ala Pro Lys Gly Glu
   340 345 350
   Phe Tyr Asn Tyr Ser Gly Cys Gly Asn Thr Phe Asn Cys Asn His Pro
   355 360 365
   Val Val Arg Gln Phe Ile Val Asp Cys Leu Arg Tyr Trp Val Thr Glu
   370 375 380
   Met His Val Asp Gly Phe Arg Phe Asp Leu Ala Ser Ile Met Thr Arg
   385 390 395 400
   Gly Ser Ser Leu Trp Asp Pro Val Asn Val Tyr Gly Ala Pro Ile Glu
   405 410 415
   Gly Asp Met Ile Thr Thr Gly Thr Pro Leu Val Thr Pro Pro Leu Ile
               420                 425                 430
   Asp Met Ile Ser Asn Asp Pro Ile Leu Gly Gly Val Lys Leu Ile Ala
           435                 440                 445
   Glu Ala Trp Asp Ala Gly Gly Leu Tyr Gln Val Gly Gln Phe Pro His
       450                 455                 460
   Trp Asn Val Trp Ser Glu Trp Asn Gly Lys Tyr Arg Asp Ile Val Arg
   465                 470                 475                 480
   Gln Phe Ile Lys Gly Thr Asp Gly Phe Ala Gly Gly Phe Ala Glu Cys
                   485                 490                 495
   Leu Cys Gly Ser Pro His Leu Tyr Gln Ala Gly Gly Arg Lys Pro Trp
               500                 505                 510
   His Ser Ile Asn Phe Val Cys Ala His Asp Gly Phe Thr Leu Ala Asp
           515                 520                 525
   Leu Val Thr Tyr Asn Lys Lys Tyr Asn Leu Pro Asn Gly Glu Asn Asn
       530                 535                 540
   Arg Asp Gly Glu Asn His Asn Leu Ser Trp Asn Cys Gly Glu Glu Gly
   545                 550                 555                 560
   Glu Phe Ala Arg Leu Ser Val Lys Arg Leu Arg Lys Arg Gln Met Arg
                   565                 570                 575
   Asn Phe Phe Val Cys Leu Met Val Ser Gln Gly Val Pro Met Phe Tyr
               580                 585                 590
   Met Gly Asp Glu Tyr Gly His Thr Lys Gly Gly Asn Asn Asn Thr Tyr
           595                 600                 605
   Cys His Asp Ser Tyr Val Asn Tyr Phe Arg Trp Asp Lys Lys Glu Gln
       610                 615                 620
   Tyr Ser Glu Leu His Arg Phe Cys Cys Leu Met Thr Lys Phe Arg Lys
   625                 630                 635                 640
   Glu Cys Glu Gly Leu Gly Leu Glu Asp Phe Pro Thr Ala Lys Arg Leu
                   645                 650                 655
   Gln Trp His Gly His Gln Pro Gly Lys Pro Asp Trp Ser Glu Asn Ser
               660                 665                 670
   Arg Phe Val Ala Phe Ser Met Lys Asp Glu Arg Gln Gly Glu Ile Tyr
           675                 680                 685
   Val Ala Phe Asn Thr Ser His Leu Pro Ala Val Val Glu Leu Pro Glu
       690                 695                 700
   Arg Ala Gly Arg Arg Trp Glu Pro Val Val Asp Thr Gly Lys Pro Ala
   705 710 715 720
   Pro Tyr Asp Phe Leu Thr Asp Asp Leu Pro Asp Arg Ala Leu Thr Ile
   725 730 735
   His Gln Phe Ser His Phe Leu Tyr Ser Asn Leu Tyr Pro Met Leu Ser
   740 745 750
   Tyr Ser Ser Val Ile Leu Val Leu Arg Pro Asp Val
   755 760

<210> 4
<211> 24
<212> DNA
<213> Artificial Sequence
<220>
<223> primer

>400> 4

<210> 5
<211> 31
<212> DNA
<213> Artificial Sequence
<220>
<223> primer

<400> 5
   GCCATTTCAA CCGTTCTGAA GTCGGGAAGT C 31

<210> 6
<211> 2437
<212> DNA
<213> Triticum aestivum L. cv. Florida
<220>
<222> CDS
<223> (16) ... (2304)

<400> 6
   GAATTCGGCA CGAGG CCG GCG CCG CGC CTG CGA CGG TGG CGG CCC AAT GCG 51
   Pro Ala Pro Arg Leu Arg Arg Trp Arg Pro Asn Ala
   1 5 10
   ACG GCG GGG AAG GGG GTC GGC GAG GTC TGC GCC GCG GTT GTC GAG GTG 99
   Thr Ala Gly Lys Gly Val Gly Glu Val Cys Ala Ala Val Val Glu Val
            15                       20                       25
   GCG ACG AAG GCC GAG GAT GAG GGG GAG GAG GAC GAG CCG GTG GCG GAG        147
   Ala Thr Lys Ala Glu Asp Glu Gly Glu Glu Asp Glu Pro Val Ala Glu
        30                       35                       40
   GAC AGG TAC GCG CTC GGC GGC GCG TGC AGG GTG CTC GCC GGA ATG CCC        195
   Asp Arg Tyr Ala Leu Gly Gly Ala Cys Arg Val Leu Ala Gly Met Pro
    45                      50                       55                   60
   ACG CCG CTG GGC GCC ACC GCG CTC GCC GGC GGG GTC AAT TTC GCC GTC        243
   Thr Pro Leu Gly Ala Thr Ala Leu Ala Gly Gly Val Asn Phe Ala Val
                        65                       70                   75
   TAC TCC GGC GGA GCC ACA GCC GCG GCG CTC TGC CTC TTC ACG CCA GAA        291
   Tyr Ser Gly Gly Ala Thr Ala Ala Ala Leu Cys Leu Phe Thr Pro Glu
                    80                       85                   90
   GAT CTC AAG GCG GAT AGG GTG ACG GAG GAG GTT CCC CTT GAC CCC CTG        339
   Asp Leu Lys Ala Asp Arg Val Thr Glu Glu Val Pro Leu Asp Pro Leu
            95                      100                      105
   ATG AAT CGG ACT GGG AAC GTA TGG CAT GTC TTC ATC GAA GGC GAG CTG        387
   Met Asn Arg Thr Gly Asn Val Trp His Val Phe Ile Glu Gly Glu Leu
       110                      115                      120
   CAG GAC ATG CTT TAC GGG TAC AGG TTC GAC GGC ACC TTT GCT CCT CAC        435
   Gln Asp Met Leu Tyr Gly Tyr Arg Phe Asp Gly Thr Phe Ala Pro His
   125                      130                      135                 140
   TGC GGG CAC TAC CTT GAT GTT TCC AAT GTC GTG GTG GAT CCT TAT GCT        483
   Cys Gly His Tyr Leu Asp Val Ser Asn Val Val Val Asp Pro Tyr Ala
                       145                      150                 155
   AAG GCA GTG ATA AGC CGA GGG GAG TAT GGT GTT CCG GCG CGT GGT AAC        531
   Lys Ala Val Ile Ser Arg Gly Glu Tyr Gly Val Pro Ala Arg Gly Asn
                   160                      165                      170
   AAT TGC TGG CCT CAG ATG GCT GGC ATG ATC CCT CTT CCA TAT AGC ACG        579
   Asn Cys Trp Pro Gln Met Ala Gly Met Ile Pro Leu Pro Tyr Ser Thr
           175                      180                      185
   TTT GAT TGG GAA GGC GAC CTA CCT CTA AGA TAT CCT CAA AAG GAC CTG        627
   Phe Asp Trp Glu Gly Asp Leu Pro Leu Arg Tyr Pro Gln Lys Asp Leu
       190                      195                      200
   GTA ATA TAT GAG ATG CAC TTG CGT GGA TTC ACG AAG CAT GAT TCA AGC        675
   Val Ile Tyr Glu Met His Leu Arg Gly Phe Thr Lys His Asp Ser Ser
   205                      210                      215                 220
   AAT GTA GAA CAT CCC GGT ACT TTC ATT GGG GCT GTG TCG AAG CTT GAC        723
   Asn Val Glu His Pro Gly Thr Phe Ile Gly Ala Val Ser Lys Leu Asp
                       225                 230                 235
   TAT TTG AAG GAG CTT GGA GTT AAT TGT ATT GAG TTA ATG CCC TGC CAT       771
   Tyr Leu Lys Glu Leu Gly Val Asn Cys Ile Glu Leu Met Pro Cys His
                   240                 245                 250
   GAG TTC AAC GAG CTG GAG TAC TCA ACC TCT TCT TCC AAG ATG AAC TTT       819
   Glu Phe Asn Glu Leu Glu Tyr Ser Thr Ser Ser Ser Lys Met Asn Phe
               255                 260                 265
   TGG GGA TAT TCT ACC ATA AAC TTC TTT TCA CCA ATG ACG AGA TAC ACA       867
   Trp Gly Tyr Ser Thr Ile Asn Phe Phe Ser Pro Met Thr Arg Tyr Thr
       270                 275                 280
   TCA GGC GGG ATA AAA AAC TGT GGG CGT GAT GCC ATA AAT GAG TTC AAA       915
   Ser Gly Gly Ile Lys Asn Cys Gly Arg Asp Ala Ile Asn Glu Phe Lys
   285                 290                 295                 300
   ACT TTT GTA AGA GAG GCT CAC AAA CGG GGA ATT GAG GTG ATC CTG GAT       963
   Thr Phe Val Arg Glu Ala His Lys Arg Gly Ile Glu Val Ile Leu Asp
                   305                 310                 315
   GTT GTC TTC AAC CAT ACA GCT GAG GGT AAT GAG AAT GGT CCA ATA TTA      1011
   Val Val Phe Asn His Thr Ala Glu Gly Asn Glu Asn Gly Pro Ile Leu
               320                 325                 330
   TCA TTT AGG GGG GTC GAT AAT ACT ACA TAC TAT ATG CTT GCA CCC AAG      1059
   Ser Phe Arg Gly Val Asp Asn Thr Thr Tyr Tyr Met Leu Ala Pro Lys
               335                 340                 345
   GGA GAG TTT TAT AAC TAT TCT GGC TGT GGG AAT ACC TTC AAC TGT AAT      1107
   Gly Glu Phe Tyr Asn Tyr Ser Gly Cys Gly Asn Thr Phe Asn Cys Asn
       350                 355                 360
   CAT CCT GTG GTT CGT CAA TTC ATT GTA GAT TGT TTA AGA TAC TGG GTG      1155
   His Pro Val Val Arg Gln Phe Ile Val Asp Cys Leu Arg Tyr Trp Val
   365                 370                 375                 380
   ACG GAA ATG CAT GTT GAT GGT TTT CGT TTT GAT CTT GCA TCC ATA ATG      1203
   Thr Glu Met His Val Asp Gly Phe Arg Phe Asp Leu Ala Ser Ile Met
                   385                 390                 395
   ACC AGA GGT TCC AGT CTG TGG GAT CCA GTT AAC GTG TAT GGA GCT CCA      1251
   Thr Arg Gly Ser Ser Leu Trp Asp Pro Val Asn Val Tyr Gly Ala Pro
               400                 405                 410
   ATA GAA GGT GAC ATG ATC ACA ACA GGG ACA CCT CTT GTT ACT CCA CCA      1299
   Ile Glu Gly Asp Met Ile Thr Thr Gly Thr Pro Leu Val Thr Pro Pro
           415                 420                 425
   CTT ATT GAC ATG ATC AGC AAT GAC CCA ATT CTT GGA GGC GTC AAG CTC      1347
   Leu Ile Asp Met Ile Ser Asn Asp Pro Ile Leu Gly Gly Val Lys Leu
   430 435 440
   GTT GCT GAA GCA TGG GAT GCA GGA GGC CTC TAT CAA GTA GGT CAA TTC 1395
   Val Ala Glu Ala Trp Asp Ala Gly Gly Leu Tyr Gln Val Gly Gln Phe
   445 450 455 460
   CCT CAC TGG AAT GTT TGG TCT GAG TGG AAT GGG AAG TAC CGG GAC ATT 1443
   Pro His Trp Asn Val Trp Ser Glu Trp Asn Gly Lys Tyr Arg Asp Ile
   465 470 475
   GTG CGT CAA TTC ATT AAA GGC ACT GAT GGA TTT GCT GGT GGT TTT GCC 1491
   Val Arg Gln Phe Ile Lys Gly Thr Asp Gly Phe Ala Gly Gly Phe Ala
   480 485 490
   GAA TGT CTT TGT GGA AGT CCA CAC CTA TAC CAG GCA GGA GGA AGG AAA 1539
   Glu Cys Leu Cys Gly Ser Pro His Leu Tyr Gln Ala Gly Gly Arg Lys
   495 500 505
   CCT TGG CAC AGT ATC AAC TTT GTA TGT GCA CAC GAT GGA TTT ACA CTG 1587
   Pro Trp His Ser Ile Asn Phe Val Cys Ala His Asp Gly Phe Thr Leu
   510 515 520
   GCT GAT TTG GTA ACA TAT AAT AAC AAG TAC AAT TTA CCA AAT GGG GAG 1635
   Ala Asp Leu Val Thr Tyr Asn Asn Lys Tyr Asn Leu Pro Asn Gly Glu
   525 530 535 540
   AAC AAC AGA GAT GGA GAA AAT CAC AAT CTT AGC TGG AAT TGT GGG GAG 1683
   Asn Asn Arg Asp Gly Glu Asn His Asn Leu Ser Trp Asn Cys Gly Glu
   545 550 555
   GAA GGA GAA TTC GCA AGA TTG TCT GTC AAA AGA TTG AGG AAG AGG CAG 1731
   Glu Gly Glu Phe Ala Arg Leu Ser Val Lys Arg Leu Arg Lys Arg Gln
   560 565 570
   ATG CGC AAT TTC TTT GTT TGT CTC ATG GTT TCT CAA GGA GTT CCA ATG 1779
   Met Arg Asn Phe Phe Val Cys Leu Met Val Ser Gln Gly Val Pro Met
   575 580 585
   TTC TAC ATG GGT GAT GAA TAT GGC CAC ACA AAA GGG GGC AAC AAC AAT 1827
   Phe Tyr Met Gly Asp Glu Tyr Gly His Thr Lys Gly Gly Asn Asn Asn
   590 595 600
   ACA TAC TGC CAT GAT TCT TAT GTC AAT TAT TTT CGC TGG GAT AAA AAA 1875
   Thr Tyr Cys His Asp Ser Tyr Val Asn Tyr Phe Arg Trp Asp Lys Lys
   605 610 615 620
   GAA CAA TAC TCT GAC TTG CAC CGA TTC TGT TGC CTC ATG ACC AAA TTC 1923
   Glu Gln Tyr Ser Asp Leu His Arg Phe Cys Cys Leu Met Thr Lys Phe
   625 630 635
   CGC AAG GAG TGC GAG GGT CTT GGC CTT GAG GAT TTT CCA ACG GCC GAA 1971
   Arg Lys Glu Cys Glu Gly Leu Gly Leu Glu Asp Phe Pro Thr Ala Glu
   640 645 650
   CGG CTG CAG TGG CAT GGT CAT CAG CCT GGG AAG CCT GAT TGG TCT GAG 2019
   Arg Leu Gln Trp His Gly His Gln Pro Gly Lys Pro Asp Trp Ser Glu
   655 660 665
   AAT AGC CGA TTC GTT GCC TTT TCC ATG AAA GAT GAA AGA CAG GGC GAG 2067
   Asn Ser Arg Phe Val Ala Phe Ser Met Lys Asp Glu Arg Gln Gly Glu
   670 675 680
   ATC TAT GTG GCC TTC AAC ACC AGC CAC TTA CCG GCC GTT GTT GAG CTC 2115
   Ile Tyr Val Ala Phe Asn Thr Ser His Leu Pro Ala Val Val Glu Leu
   685 690 695 700
   CCG GAG CGC ACA GGG CGC CGG TGG GAA CCG GTG GTG GAC ACA GGC AAG 2163
   Pro Glu Arg Thr Gly Arg Arg Trp Glu Pro Val Val Asp Thr Gly Lys
   705 710 715
   CCA GCA CCA TAC GAC TTC CTC ACT GAC GAC TTA CCT GAT CGC GCT CTC 2211
   Pro Ala Pro Tyr Asp Phe Leu Thr Asp Asp Leu Pro Asp Arg Ala Leu
   720 725 730
   ACC ATA CAC CAG TTC TCT CAT TTC CTC AAC TCC AAC CTC TAC CCC ATG 2259
   Thr Ile His Gln Phe Ser His Phe Leu Asn Ser Asn Leu Tyr Pro Met
   735 740 745
   CTC AGC TAC TCA TCG GTC ATC CTA GTA TTG CGC CCT GAT GTT TGAGAGGCGG 2311
   Leu Ser Tyr Ser Ser Val Ile Leu Val Leu Arg Pro Asp Val
   750 755 760
   ATATACAGTA AATAATATGT ATATATGTAG TCCTTTGGCG TATTATCAGT GTGCACAATT 2371
   GCTCTATTGC CAATGATCTA TTCGATCCAC AGATACATGT GCAAAAAAAA AAAAAAAAAA 2431
   CTCGAG 2437

<210> 7
<211> 764
<212> PRT
<213> Triticum aestivum L. cv. Florida

<400> 7

<210> 8
<211> 20
<212> DNA
<213> Artificial Sequence
<220>
<223> primer

<400> 8
   GCTTTACGGG TACAGGTTCG 20

<210> 9
<211> 18
<212> DNA
<213> Artificial Sequence
<220>
<223> primer

<400> 9
   AAT TCC CCG TTT GTG AGC 18

<210> 10
<211> 51
<212> DNA
<213> Artificial Sequence
<220>
<223> primer

<400> 10
   GCGGTACCTC TAGAAGGAGA TATACATATG GCGGAGGACA GGTACGCGCT C 51

<210> 11
<211> 33
<212> DNA
<213> Artificial Sequence
<220>
<223> primer

<400> 11
   GCTCGAGTCG ACTCAAACAT CAGGGCGCAA TAC 33